# FASCICULE DE BREVET EUROPEEN

(11) **EP 3 689 327 B1**
(45) Date de publication et mention de la délivrance du brevet: **12.03.2025**
(21) Numéro de dépôt: 20161781.8
(22) Date de dépôt: 24.01.2019
(51) Int. Cl.: A61K 8/73, A61K 31/728, A61K 31/737, A61P 17/02, A61P 25/28, A61P 27/02, A61P 17/00, A61P 19/02, A61Q 19/08, A61P 11/00

(54) **COMPOSITION PHARMACEUTIQUE DANS LE TRAITEMENT DE LÉSIONS PULMONAIRES**
PHARMAZEUTISCHE ZUSAMMENSETZUNG ZUR BEHANDLUNG VON LUNGENLÄSIONEN
PHARMACEUTICAL COMPOSITION FOR THE TREATMENT OF PULMONARY LESIONS

(43) Date de publication de la demande: 05.08.2020
(62) Demande divisionnaire de: 19305095.2
(73) Titulaire: Organes Tissus Régénération Réparation Remplacement, 75001 Paris (FR); Barritault, Denis, 75001 Paris (FR)
(72) Inventeur: BARRITAULT, Denis, 75001 PARIS (FR)
(74) Mandataire: Novagraaf Technologies

(56) Documents cités:
- EP-A1- 3 097 922
- EP-A2- 2 260 853
- FR-A1- 2 861 308
- US-A1- 2018 125 880
- BARRITAULT DENIS ET AL: "RGTA or ReGeneraTing Agents mimic heparan sulfate in regenerative medicine: from concept to curing patients", GLYCOCONJUGATE JOURNAL, CHAPMAN & HALL, BOSTON, vol. 34, no. 3, 7 December 2016 (2016-12-07), pages 325 - 338, XP036255501, ISSN: 0282-0080, [retrieved on 20161207], DOI: 10.1007/S10719-016-9744-5
- ANONYMOUS: "OTR3 Organ, tissue, Regeneration, Repair, Replacement", 1 January 2017 (2017-01-01), XP055607820, Retrieved from the Internet <URL:http://www.pharmetikon.com/wp-content/uploads/2017/02/Presentation-CACIPLIQ-04.2016_v-distributeurs.pdf> [retrieved on 20190723]
- TIWARI SANJAY ET AL: "Modified hyaluronic acid based materials for biomedical applications", INTERNATIONAL JOURNAL OF BIOLOGICAL MACROMOLECULES, ELSEVIER BV, NL, vol. 121, 12 October 2018 (2018-10-12), pages 556 - 571, XP085535409, ISSN: 0141-8130, DOI: 10.1016/J.IJBIOMAC.2018.10.049
- GERARD M. TURINO ET AL: "Hyaluronan in Respiratory Injury and Repair", AMERICAN JOURNAL OF RESPIRATORY AND CRITICAL CARE MEDICINE., vol. 167, no. 9, 1 May 2003 (2003-05-01), US, pages 1169 - 1175, XP055598228, ISSN: 1073-449X, DOI: 10.1164/rccm.200205-449PP

## Description

### Domaine technique

La présente invention telle que définie dans les revendications se rapporte à une composition pharmaceutique ou dermatologique et son utilisation comme médicament ou dispositif médical.

La présente invention se rapporte également à une composition cosmétique ou dermatologique, ainsi qu'à une trousse de soin comprenant la composition de l'invention.

La présente invention se rapporte également à une composition cosmétique ou dermatologique anti-âge, ainsi qu'à une trousse de soin comprenant la composition de l'invention.

La présente invention se rapporte également à une composition cosmétique, dermatologique hydratante, ainsi qu'à une trousse de soin comprenant la composition de l'invention.

La présente invention se rapporte également à une composition ophtalmologique, notamment une composition ophtalmologique hydratante de la cornée, ainsi qu'à une trousse de soin comprenant la composition de l'invention.

La présente invention se rapporte également à une composition cosmétique ou dermatologique antirides, ainsi qu'à une trousse de soin comprenant la composition de l'invention.

La présente invention se rapporte également à une composition cosmétique ou dermatologique pour son utilisation dans le traitement du vieillissement de la peau, pour l'amélioration de l'aspect de la peau, ainsi qu'à une trousse de soin comprenant la composition de l'invention.

La présente invention trouve une application notamment dans les domaines cosmétiques, pharmaceutiques et vétérinaires.

La présente invention trouve une application notamment dans le domaine articulaire pharmaceutique et vétérinaire, notamment pour l'amélioration et pour faciliter le mouvement et la récupération des fonctions motrice

La présente invention trouve une application notamment dans le domaine pharmaceutique et vétérinaire du mouvement des vertèbres, notamment pour l'amélioration et pour faciliter le mouvement et la récupération des fonctions motrices.

La présente invention trouve une application dans le domaine dentaire notamment dans l'amélioration des gencives et des muqueuses buccales, les fibroses, par exemple les fibroses des muscles, les adhésions, les fibroses du système digestif, les fibroses liés à la chirurgie, les fibroses du cœur, les fibroses du cerveau, comme anti oxydant, par exemple pour le traitement stress oxydatif

Dans la description ci-dessous, les références entre crochet [ ] renvoient à la liste des références présentée à la fin du texte.

### Etat de la technique

Plus qu'une enveloppe extérieure, la peau est un organe à part entière qui joue un rôle essentiel, non seulement dans la protection du corps contre les agressions extérieures, mais aussi sur le plan esthétique et émotionnel. La peau respire, elle est sensible aux agressions extérieures et elle réagit à notre état de santé général ainsi qu'à l'équilibre hormonal.

La peau humaine est constituée de deux compartiments : l'épiderme et le derme. L'hypoderme ou tissu sous cutanée conjonctif d'épaisseur très variable est souvent fonctionnellement rattaché à la peau et est constitué essentiellement de tissus adipeux très vascularisé et innervé et servant d'interface aux muscles et tendons sous-jacents. S'insèrent les glandes sudoripares, sébacées et les phanères, poils et ongles.

Une peau en bonne santé est une peau bien hydratée. Elle est douce, ferme, souple et élastique. Le derme est constitué d'une structure cellulaire dont la mission principale est d'assurer l'hydratation de notre peau. Dans l'épiderme, des lamelles de cellules cornées, composées de kératine protectrice en forme de mille feuilles, servent de barrage pour retenir l'eau et filtrer son évaporation. La peau est constituée d'environ 70% d'eau, soit 1/5 de l'eau stockée dans notre corps. L'eau circule du derme vers l'épiderme. Un coussin cellulaire constitué de corps gras à base de lipides, et plus particulièrement de céramides, empêche l'eau de s'évaporer. C'est lui qui détermine le facteur naturel d'hydratation de notre épiderme. Une peau bien hydratée a un taux normal d'hydratation situé entre 13% et 15%. Lorsque ce taux descend à 10%, la peau est déshydratée. Lorsque l'épiderme n'est pas correctement hydraté, par manque d'eau ou d'acides gras essentiels, la peau perd de sa souplesse et devient terne.

Les premiers signes visibles liés à l'âge apparaissent dans la texture, la souplesse, la couleur la transparence et l'apparition de rides.

Dans le domaine de l'esthétique du visage, les changements de la peau liés à l'âge et aux régulations hormonales sont classiquement répertoriés comme une atrophie, un relâchement et un engraissement de la peau. L'atrophie correspond à l'amincissement important du derme et de l'épiderme se traduisant par une diminution du nombre de couches de cellules de l'épiderme et de l'épaisseur du tissu conjonctif dermique, le relâchement de l'hypoderme et des tissus sous cutanés (graisses, muscles) se traduisant par un excès de peau et un ptosis. Le relâchement par un affaissement visible au niveau des joues et du menton ainsi que sous les paupières, quant à l'engraissement lié à une augmentation du poids avec l'âge, il s'observe par un gonflement du bas du visage et du cou.

Ces signes de vieillissement liés au temps peuvent être accélérés et amplifiés par le style de vie comme notamment l'abus de tabac, d'alcool ou l'exposition au soleil. Ils sont aussi souvent accompagnés d'une sécheresse et une rugosité de la peau associés à une perte de l'élasticité résultant en une altération de la qualité du tissu cutanée. La peau forme des plis sous des pressions mécaniques et met plus de temps à récupérer et retrouver son état initial. Ces signes visuels n'étant que le reflet extérieur de ces changements internes liés à l'âge de l'organisation de la peau.

La diminution de l'épaisseur de l'épiderme avec l'âge et se traduit par une réduction de la multiplication des cellules de la couche basale, par une réduction du nombre de couches de cellules épidermiques, une altération de la matrice extracellulaire quantitatif, structural, organisationnelle et fonctionnelle tant sur les protéines que sur les Glycosaminoglycanes (GAGs) et protéoglycanes (PGs) et l'apparition des rides.

Parmi ces (GAG) l'Acide Hyaluronique (AH) joue un rôle central dans le maintien de l'hydratation à tous les niveaux des couches de la peau et sa diminution est corrélée avec l'apparition des rides et avec les héparanes sulfates (HS) dans la régulation de l'équilibre naturel de l'homéostasie tissulaire en assurant un quadruple rôle comme élément de stabilisation, de protection de l'architecture matriciel, de stockage et de régulateur de la biodisponibilité des facteurs de communication cellulaire dans le micro environnement tant dans l'épiderme que dans le derme ou l'hypoderme.

Dans le but de réduire les effets inesthétiques liés aux altérations et vieillissement de la peau, de nombreuses approches ont visé à prévenir, retarder, améliorer des différents éléments tant protéiques que GAGs présents dans la peau. **Il** peut s'agir notamment d'une approche afin d'essayer d'induire une augmentation de leur synthèse, en retardant leur dégradation, en introduisant des produits capables de se substituer aux produits endogènes mais plus résistants à la dégradation, ou l'introduction de stimulants de la synthèse des protéines de la matrice tels les collagènes ou l'élastine améliore également les performances de la peau au niveau mécanique et esthétique. Une autre possibilité est l'utilisation d'agent tension actifs a également des effets immédiatement visibles sur la peau en réduisant les rides et ridules et lissant la peau mais ces effets sont à très court termes et superficiels.

**Il** existe dans l'état de la technique de nombreuses compositions cosmétiques, principes actifs pour le traitement du vieillissement de la peau. Toutefois, aucune de ces compositions, ou aucun de ces principes actifs ne permet d'agir durablement sur les signes du vieillissement et/ou de permettant de restaurer/améliorer naturellement la structure de la peau.

**Il** existe donc un réel besoin dans l'état de la technique de trouver un composé et/ou une composition permettant de traiter efficacement le vieillissement cutané et/ou améliorer l'aspect de la peau de manière durable et/ou améliorant la structure de la peau.

**Il** existe également dans l'état de la technique de nombreuses compositions cosmétiques, principes actifs pour le traitement du vieillissement de la peau dont l'effet et/ou le bénéfice est limité dans le temps, notamment de par sa vitesse d'élimination naturelle et/ou liée à l'environnement limitant ainsi la durée de l'effet qui est liée à sa présence sur la zone à traiter et/ou sur la peau.

Il existe donc un réel besoin dans l'état de la technique de trouver un composé et/ou une composition dont l'effet et/ou le bénéfice est prolongé dans le temps, notamment un composé et/ou une composition susceptible permettant de traiter efficacement le vieillissement cutané et/ou améliorer l'aspect de la peau de manière durable et/ou améliorant la structure de la peau.

En outre, les compositions et/ou principes actifs connus ne permettent pas de restaurer et/ou améliorer efficacement la structure de la peau. En particulier, les compositions et/ou principes actifs connus ne permettent pas de restaurer et/ou d'améliorer la structure de la peau et/ou de prévenir efficacement le vieillissement de la peau.

Par ailleurs, les compositions et/ou principes actifs connus nécessitent, pour une efficacité relative, un nombre important d'applications et/ou une durée de traitement et/ou une fréquence d'application, notamment sur la peau, élevé. Le nombre important d'applications et/ou la durée importante/longue de traitement peut éventuellement provoquer une sensibilisation de la peau et entrainer d'éventuelles réactions allergiques.

Il existe donc un réel besoin dans l'état de la technique de trouver un composé et/ou une composition permettant de traiter efficacement le vieillissement cutané et/ou améliorer l'aspect de la peau, en particulier permettant un nombre limité d'application afin d'obtenir un effet, en particulier antiâge et/ou une amélioration de l'aspect de la peau.

De plus, les compositions et/ou principes actifs connus ont, généralement un effet superficiel, par exemple uniquement temporaire, par exemple pendant quelques heures, ne permettant pas un effet durable et/ou structurelle. En outre, les compositions et/ou principes actifs connus ne permettent pas de traiter et/ou compenser les mécanismes et/ou défauts à l'origine du vieillissement cutané.

Par exemple, l'acide hyaluronique est utilisé dans le domaine cosmétique comme composé hydratant et également dans le domaine médical, par exemple pour le traitement de sécheresse vaginale ou vulvaire. L'acide hyaluronique est utilisé notamment par injection, par exemple au niveau des lèvres vaginales afin de regonfler et réhydrater la peau et redonner du volume aux grandes lèvres qui peuvent de nouveau protéger la vulve et l'ouverture du vagin et éviter également les démangeaisons associées à cette sécheresse. L'acide hyaluronique est également utilisé en injection pour le traitement de la sécheresse oculaire, la sécheresse des cheveux et pour la réhydratation des follicules pileux par exemple sous la forme de gel ou en micro-injection par exemple en mésothérapie seul ou avec des cofacteurs nutritifs ou protecteurs. L'acide hyaluronique peut être également utilisé en injection pour améliorer la vie sexuelle des hommes par magnification de leur gland. Des exemples d'applications et/ou utilisations de l'acide hyaluronique sont illustrés notamment dans les documents suivant Modified hyaluronic acid based materials for biomedical applications. Tiwari S, Bahadur P.Int J Biol Macromol. 2019 Jan;121 :556-571. doi: 10.1016/j.ijbiomac.2018.10.049. Epub 2018 Oct 12. Review [1]; Hyaluronic acid, a promising skin rejuvenating biomedicine: A review of recent updates and pre-clinical and clinical investigations on cosmetic and nutricosmetic effects. Bukhari SNA, Roswandi NL, Waqas M, Habib H, Hussain F, Khan S, Sohail M, Ramli NA, Thu HE, Hussain Z. Int J Biol Macromol. 2018 Dec; 120(Pt B):1682-1695. doi: 10.1016/j.ijbiomac.2018.09.188. doi:10.1016/j.ijbiomac.2018.09.188 [2], et Reduction of postoperative adhesion development. Diamond MP. Fertil Steril. 2016 Oct;106(5):994-997.e1. doi: 10.1016/j.fertnstert.2016.08.029. Epub 2016 Sep 10. Review [3]. Toutefois, bien que présentant de nombreuses applications, l'acide hyaluronique est utile en injection ce qui implique la nécessité d'un médecin et un acte médical précis ne pouvant être effectué quotidiennement par un utilisateur.

Il existe donc un réel besoin dans l'état de la technique de trouver un composé et/ou une composition permettant de traiter efficacement la sécheresse des tissus, notamment cutané, en particulier un composé et/ou une composition, par application locale, par exemple par simple application sur le tissu, par exemple cutané.

Il existe également un réel besoin dans l'état de la technique de trouver un composé et/ou une composition permettant de traiter efficacement le vieillissement de la peau et/ou permettant une amélioration de l'aspect de la peau, par application locale, par exemple par simple application sur le tissu, par exemple cutané.

Il est également connu qu'avec le temps, les tissus, notamment la peau, subissent une évolution lente et irréversible conduisant à des changements anatomiques, histologiques et fonctionnels.

Il existe dans l'état de la technique de nombreuses compositions cosmétiques, principes actifs pour le traitement du vieillissement de la peau. Toutefois, aucune de ces compositions, ou aucun de ces principes actifs ne permet d'agir et/ou d'avoir un effet significatif sur le ou les mécanismes impliqués dans les changements anatomiques, histologiques et/ou fonctionnels des tissus, notamment de la peau.

Il existe donc un réel besoin dans l'état de la technique de trouver un composé et/ou une composition permettant d'agir et/ou d'avoir un effet significatif sur le ou les mécanismes impliqués dans les changements anatomiques, histologiques et/ou fonctionnels des tissus, notamment de la peau.

Il existe dans l'état de la technique des composés utilisés dans le domaine thérapeutique susceptibles d'améliorer l'environnement tissulaire, par exemple des polymères biocompatibles, en particulier le RGTA est connu de l'homme de l'art pour avoir un effet sur un processus de réparation et de régénération tissulaire par une action sur la matrice extracellulaire, notamment une éventuelle protection d'éléments protéiques du microenvironnement cellulaire et tissulaire. Ils sont également connus pour des propriétés également apaisantes et anti douleurs, éventuellement des activités anti-fibrose (document brevet US06689741, US2014301972A1 [4], Reversal of abnormal collagen production in Crohn's disease intestinal biopsies treated with regenerating agents. Alexakis C, Caruelle JP, Sezeur A, Cosnes J, Gendre JP, Mosnier H, Beaugerie L, Gallot D, Malafosse M, Barritault D, Kern P. Gut. 2004 Jan;53(1):85-90 [5]), antioxydantes (Insights on a new path of pre-mitochondrial apoptosis regulation by a glycosaminoglycan mimetic. Yue XL, Lehri S, Li P, Barbier-Chassefière V, Petit E, Huang QF, Albanese P, Barritault D, Caruelle JP, Papy-Garcia D, Morin C. Cell Death Differ. 2009 May;16(5):770-81. doi: 10.1038/cdd.2009.9 [6], Differential effect triggered by a heparan mimetic of the RGTA family preventing oral mucositis without tumor protection. Mangoni M, Yue X, Morin C, Violot D, Frascogna V, Tao Y, Opolon P, Castaing M, Auperin A, Biti G, Barritault D, Vozenin-Brotons MC, Deutsch E, Bourhis J. Int J Radiat Oncol Biol Phys. 2009 Jul 15;74(4):1242-50. doi: 10.1016/j.ijrobp.2009.03.006 [7]), cicatrisantes (RGTA OTR4120, a heparan sulfate mimetic, is a possible long-term active agent to heal burned skin. Garcia-Filipe S, Barbier-Chassefiere V, Alexakis C, Huet E, Ledoux D, Kerros ME, Petit E, Barritault D, Caruelle JP, Kern P J Biomed Mater Res A. 2007 Jan;80(1):75-8) favorisant la cicatrisation cutanée et pour le traitement de la douleur (Document brevet EP1677807 [9])

Ces activités peuvent être notamment dues à une capacité pour ces composés à inhiber certaines glycanases, par exemple l'héparanase, héparitinases, les Chondroitinases et les Hyaluronidases (Heparin-like synthetic polymers, named RGTAs, mimic biological effects of heparin in vitro. Rouet V, Meddahi-Pellé A, Miao HQ, Vlodavsky I, Caruelle JP, Barritault D. J Biomed Mater Res A. 2006 Sep 15;78(4):792-7 [10], EP1677807 [9], Document brevet US06689741, US2014301972A1 [4]), à inhiber l'élastase (FGF protection and inhibition of human neutrophil elastase by carboxymethyl benzylamide sulfonate dextran derivatives. Meddahi A, Lemdjabar H, Caruelle JP, Barritault D, Hornebeck W. Int J Biol Macromol. 1996 Feb;18 (1-2):141-5 [11], des collagénases, la plasmine (Human plasmin enzymatic activity is inhibited by chemically modified dextrans. Ledoux D, Papy-Garcia D, Escartin Q, Sagot MA, Cao Y, Barritault D, Courtois J, Hornebeck W, Caruelle JP. J Biol Chem. 2000 Sep 22;275(38):29383-90 [12]) et l'activateur du plasminogène ou la calpaïne (Heparan sulfate mimetics modulate calpain activity during rat Soleus muscle regeneration. Zimowska M, Szczepankowska D, Streminska W, Papy D, Tournaire MC, Gautron J, Barritault D, Moraczewski J, Martelly I. J Cell Physiol. 2001 Aug;188(2):178-87 [13]), à protéger au niveau des sites sur lesquels ils se fixent (sites décrits dans la littérature comme site de fixation des héparanes sulfates ou « Heparan/heparin binding sites) des protéines matricielles (des facteurs de croissance et de communication cellulaire, à savoir le VEGF et le TGFbeta (RGTA® or ReGeneraTing Agents mimic heparan sulfate in regenerative medicine: from concept to curing patients. Barritault D, Gilbert-Sirieix M, Rice KL, Siñeriz F, Papy-Garcia D, Baudouin C, Desgranges P, Zakine G, Saffar JL, van Neck J. Glycoconj J. 2017 Jun;34(3):325-338. doi: 10.1007/s10719-016-9744-5 [14].

Toutefois, les RGTA sont utiles notamment lors de traitement de lésions importantes et, en général, reste des composés couteux qui ne peuvent être utilisé de manière usuelle, notamment en cosmétique.

Il existe donc un réel besoin de trouver un nouveau composé, une nouvelle composition, traitement permettant de traiter le vieillissement des tissus, notamment de la peau et/ou d'augmenter l'efficacité de traitement des compositions connues tout en diminuant leur coût.

Il existe également un réel besoin dans l'état de la technique de trouver un composé et/ou une composition permettant d'agir et/ou d'avoir un effet significatif sur le ou les mécanismes impliqués dans les changements anatomiques, histologiques et/ou fonctionnels des tissus, notamment de la peau, lié au vieillissement tout en présentant un coût réduit.

### Description de l'invention

La présente invention a précisément pour but de répondre à ces besoins en fournissant une composition pharmaceutique, de préférence une composition cosmétique ou dermatologique comprenant
- un polymère biocompatible de formule générale (I) suivante

   AaXxYy (I)

   dans laquelle :
   A représente un monomère,
   X représente un groupement -R₁COOR₂ ou -R₉(C=O)R₁₀,
   Y représente un groupement O- ou N-sulfonate et répondant à l'une des formules suivante -R₃OSO₃R₄, -R₅NSO₃R₆, R₇SO₃R₈
   dans lesquelles :
      R₁, R₃, R₅ et R₉ représentent indépendamment une chaîne hydrocarbonée aliphatique, éventuellement ramifiée et/ou insaturée et qui contient éventuellement un ou plusieurs cycles aromatiques à l'exception de la benzylamine et de la benzylamine sulfonate, R₂, R₄, R₆ et R₈ représentent indépendamment un atome d'hydrogène ou un cation,
      R₇ et R₁₀ représente indépendamment une liaison, une chaîne hydrocarbonée aliphatique, éventuellement ramifiée et/ou insaturée,
      « a » représente le nombre de monomères,
      « x » représente le taux de substitution des monomères A par des groupements X,
      « y » représente le taux de substitution des monomères A par des groupements Y, et
- de l'acide hyaluronique

Avantageusement, l'inventeur a démontré de manière surprenante que l'association de polymères biocompatibles de formule générale (I) tels que définis ci-dessus, également désignés RGTA dans la présente, et d'acide hyaluronique permet de manière synergique de traiter et/ou prévenir le vieillissement de tissus biologiques.

En particulier, l'inventeur a démontré que la composition selon l'invention permet avantageusement de prévenir, réduire, éliminer ou limiter les effets de l'âge sur l'apparence de la peau, que le vieillissement soit chrono biologique ou accéléré par exemple due à des agressions extérieures, par exemple dues à l'exposition à des rayonnements, par exemple UVA, UVB et/ou rayons ionisants.

L'inventeur a également démontré de manière surprenante que les effets obtenus sont visibles et durables. En particulier, l'inventeur a démontré que la composition selon l'invention permet avantageusement une réduction visible et durable des signes et marques de vieillissement, par exemple une réduction des rides et ridules, permettant avantageusement d'améliorer l'aspect de la peau qui peut avantageusement présenter un aspect de rajeunit, une peau plus belle, par exemple une peau avec plus d'éclat, une peau plus souple, plus raffermie et également plus tendue.

En particulier, l'inventeur a démontré que la composition selon l'invention permet avantageusement de protéger les tissus, par exemple la peau et/ou les muqueuses, des agressions extérieures, par exemple par exemple dues à l'exposition à des rayonnements, par exemple UVA, UVB et/ou rayons ionisants, au froid etc. En particulier, l'inventeur a démontré que la composition selon l'invention permet avantageusement d'éviter et /ou protéger les tissus, par exemple la peau et/ou les muqueuses, de la sécheresse. Par exemple, l'inventeur a démontré que la composition selon l'invention présente avantageusement et de manière synergique des effets protecteurs et/ou hydratantes des muqueuses, permet avantageusement de réduire les effets liés à la sécheresse tissulaire.

L'inventeur a également démontré que la composition selon l'invention permet avantageusement, notamment de par une amélioration des propriétés des tissus, par exemple l'hydratation, la souplesse etc, de faciliter les mouvements articulaire et tendineux, d'améliorer les mouvements intervertébraux, notamment par l'amélioration des propriétés des disques intervertébraux et d'une manière générale favoriser les récupérations fonctionnelles des tissus.

En outre, l'inventeur a démontré que la combinaison de polymères biocompatibles de formule générale (I) avec l'acide hyaluronique selon l'invention peut utilisée/appliquée de quels que soient les tissus biologiques, par exemple par application local, par exemple par application cutané et/ou à la surface des tissus biologiques. En outre, l'inventeur a démontré que la combinaison de polymères biocompatibles de formule générale (I) avec l'acide hyaluronique selon l'invention peut également être utilisée/appliquée par injection.

L'inventeur a également démontré de manière surprenante et inattendue que la combinaison de polymères biocompatibles de formule générale (I) avec l'acide hyaluronique selon l'invention présente un fort effet synergique observé dans la récupération fonctionnelle et cicatricielle des tissus.

En outre, l'inventeur a également démontré de manière surprenante et inattendue que la combinaison de polymères biocompatibles de formule générale (I) avec l'acide hyaluronique selon l'invention a un effet synergique dans le traitement cicatriciel, par exemple le traitement des cicatrices, le traitement esthétique des cicatrices.

De plus, l'inventeur a démontré de manière surprenante que la combinaison de polymères biocompatibles de formule générale (I) avec l'acide hyaluronique selon l'invention a un effet durable dans le temps, en particulier permet de prolonger à la fois la durée d'action de la combinaison en retardant l'élimination et permet avantageusement en outre restauration de la synthèse et/ou production d'éléments biologiques, par exemple de protéines, de constituant de la matrice extracellulaire dont la production est diminuée et/ou altérée avec le vieillissement, par exemple du tissu biologique.

Dans la présente par « tissu » on entend tout tissu biologique d'un mammifère connu de l'homme du métier. Il peut s'agir par exemple du tissu conjonctif, du tissu musculaire, du tissu nerveux, du tissu osseux, cartilagineux et/ou du tissu épithélial. Il peut s'agir par exemple de tout tissu biologique de tout organe, organelle de mammifère connu de l'homme du métier. Il peut s'agir par exemple du tissu du tractus digestif, de tissus du tractus gastro-intestinal, du système digestif alimentation et excrétion, du tractus génital, du système reproducteur, du système optique, olfactif ou auditif, du système sensoriel, du système circulatoire et/ou cardiovasculaire, du système respiratoire, du système musculaire, du système locomoteur. Il peut s'agir par exemple du tissu gastrique, du tissu buccal, de la cornée, du tissu tympanique, du tissu de la cochlée, de la peau, du tissu osseux, du tissu cartilagineux, du tissu tendineux, du tissu nerveux, de la moelle épinière, de fibre nerveuse, de la rétine, des artères et/ou des vaisseaux, de tissu du système digestif rénal, urinaire et/ou de tout tissu biologique permettant le passage de liquide biologiques, par exemple de de tout tissu biologique permettant le passage de liquide biologiques connu de l'homme du métier, par exemple le canal de Schlemm ou le système lymphatique.

Dans la présente par « cicatrice » on entend toute réponse tissulaire à une agression de toute nature, durée ou intensité qui se traduit par une inflammation et la formation d'un tissu cicatriciel non identique au tissu d'origine. Il peut s'agir par exemple de toute formation de tissu cicatriciel non identique au tissu d'origine connu de l'homme du métier. Il peut s'agir par exemple de tissu cicatriciel non identique au tissu d'origine dans lequel il y a formation d'une trace et/ou fibrose, par exemple présentant des aspects et des propriétés différentes du tissus d'origine, par une forme, une souplesse, une adhésion et/ou une épaisseur.

Dans la présente par monomère on entend par exemple un monomère choisi dans le groupe comprenant les sucres, les esters, les alcools, les acides aminés ou les nucléotides.

Dans la présente, les monomères A constituent les éléments de base des polymères de formule I peuvent être identiques ou différents.

Dans la présente, les monomères A peuvent être indépendamment des monomères de formule suivante : 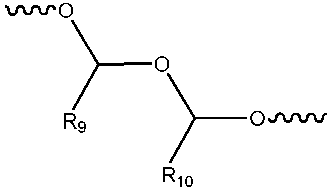 dans laquelle R₉ et R₁₀ représente indépendamment un atome d'oxygène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué, un groupement de formule -R₁₁-(X)ₙ-R₁₂ dans laquelle R₁₁ représente une chaine carbonée aliphatique en C₁ à C₄, éventuellement ramifiée et/ou insaturée, X représente un hétéroatome choisi parmi l'oxygène et l'azote, n est un entier compris de 1 à 4 et R₁₂ est un atome d'hydrogène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué.

Dans la présente, l'association de monomères peut permettre de former un squelette polymérique, par exemple un squelette polymérique de nature polyester, polyalcool, polysaccharidique, du type des acides nucléiques ou des protéines.

Dans la présente, parmi les polyesters, il peut s'agir par exemple de copolymères de biosynthèse ou synthèse chimique, par exemple des polyesters aliphatiques ou d'origine naturelle par exemple les polyhydroxyalconaotes.

Dans la présente, les polysaccharides et leurs dérivés peuvent être d'origine bactérienne, animale, fongique et/ou d'origine végétale. Il peut s'agir par exemple de polysaccharides à chaîne simple, par exemple les polyglucoses, par exemple le dextran, la cellulose, le bêta glucan, ou d'autres monomères comprenant des unités plus complexes, par exemple les xanthanes, par exemple le glucose, mannose et acide glucuronique ou encore des glucuronanes et glucoglucuronane.

Dans la présente, les polysaccharides d'origine végétale peuvent être à simple chaîne, par exemple la cellulose (glucose), les pectines (acide galacturonique), les fucanes, l'amidon ou plus complexe comme les alginates (acide galuronique et mannuronique),

Dans la présente, les polysaccharides d'origine fungique peuvent être par exemple le stéroglucane.

Dans la présente, les polysaccharides d'origine animale peuvent être par exemple les chitines ou le chitosan (glucosamine).

Dans la présente, les monomères A constituant les éléments de base des polymères de formule I peuvent être avantageusement identiques.

Dans la présente, les monomères A constituant les éléments de base des polymères de formule I peuvent être avantageusement le glucose.

Le nombre de monomères A défini dans la formule (I) par « a » peut être tel que la masse desdits polymères de formule (I) est environ entre 2 000 et 6 000 daltons, par exemple ce qui correspond à au moins 10 monomères de glucose. Par exemple masse desdits polymères de formule (I) peut environ entre 3000 daltons et 6000 daltons, par exemple ce qui correspond à 12 à 20 monomères de glucose.

Le nombre de monomères A défini dans la formule (I) par « a » peut être également tel que la masse desdits polymères de formule (I) est inférieure à environ 2 500 000 daltons (ce qui correspond à 7000 monomères de glucose). De façon avantageuse, la masse desdits polymères de formule (I) peut être comprise de 3000 à 250 000 daltons, par exemple de 3000 à 6000 daltons, ou par exemple de 20 000 à 250 000 daltons, ou par exemple de 75 000 à 150 000 daltons.

Dans la présente, dans le groupement -R₁COOR₂ représentant X, R₁ peut être un alkyle en C₁ à C₆, par exemple un méthyl, un éthyl, un butyl, un propyl, un pentyl, de préférence un groupement méthyl, et R₂ peut être une liaison, un alkyle en C₁ à C₆, par exemple un méthyl, un éthyl, un butyl, un propyl, un pentyl, un groupement R₂₁R₂₂ dans lequel R₂₁ est un anion et R₂₂ un cation choisi dans le groupe des métaux alcalins.

De préférence, le groupement X est le groupement de formule -R₁COOR₂ dans laquelle R₁ est un groupement méthyle -CH₂- et R₂ un groupement R₂₁R₂₂ dans lequel R₂₁ est un anion et R₂₂ un cation choisi dans le groupe des métaux alcalins, de préférence le groupement X est un groupement de formule -CH₂-COO⁻ou carboxyméthyl.

Dans la présente, dans le groupement -R₉(C=O)R₁₀ représentant X, R₉ peut être un alkyle en C₁ à C₆, par exemple un méthyl, un éthyl, un butyl, un propyl, un pentyl, de préférence un groupement méthyl, et R₁₀ peut être une liaison, un alkyle en C₁ à C₆, par exemple un méthyl, un éthyl, un butyl, un propyl, un pentyl, un hexyl.

Le taux de substitution de l'ensemble des monomères A par les groupements X défini dans la formule générale (I) par « x » peut être compris de 10 à 150%, de 40 à 80%, et de préférence de l'ordre de 50% ou 60%.

Dans la présente, dans le groupement répondant à l'une des formules suivantes -R₃OSO₃R₄, -R₅NSO₃R₆, -R₇SO₃R₈ et représentant le groupement Y, R₃ peut être une liaison, un alkyle en C1 à C6, par exemple un méthyl, un éthyl, un butyl, un propyl, un pentyl, de préférence un groupement méthyl, R₅ peut être une liaison, un alkyle en C₁ à C₆, par exemple un méthyl, un éthyl, un butyl, un propyl, un pentyl, de préférence un groupement méthyl, R₇ peut être une liaison, un alkyle en C₁ à C₆, par exemple un méthyl, un éthyl, un butyl, un propyl, un pentyl, de préférence un groupement méthyl, R₄, R₆ et R₈ peuvent être indépendamment un atome d'hydrogène ou un cation M⁺, par exemple M⁺ peut être un métal alcalin.

De préférence, le groupement Y est le groupement de formule -R₇SO₃R₈ dans lequel R₇ est une liaison et R₈ est un métal alcalin choisi dans le groupe comprenant le lithium, le sodium, le potassium, le rubidium et le césium. De préférence, le groupement Y est un groupement -SO₃⁻ ,-SO₃⁻ Na⁺

Le taux de substitution de l'ensemble des monomères A par les groupements Y défini dans la formule générale (I) par « y » peut être compris de 10 à 170%, de 30 à 150%, de 55 à 160%, de 55 à 85%, de 120 à 160%, et de préférence de l'ordre de 70, 140 ou 150%.

Dans la présente, la définition des taux de substitutions ci- dessus, on entend par un taux de substitution « x » de 100%, le fait que chaque monomère A du polymère de l'invention contient statistiquement un groupement X. De même, on entend par un taux de substitution « y » de 100%, le fait que chaque monomère du polymère de l'invention contient statistiquement un groupement Y. Les taux de substitution supérieurs à 100% traduisent le fait que chaque monomère porte statistiquement plus d'un groupement du type considéré ; à l'inverse, les taux de substitution inférieurs à 100% traduisent le fait que chaque monomère porte statistiquement moins d'un groupement du type considéré.

Les polymères peuvent également comprendre des groupements chimiques fonctionnels, désignés Z, différents de X et Y.

Dans la présente, les groupements Z peuvent être identiques ou différent, et peuvent indépendamment être choisis dans le groupe comprenant des acides aminés, des acides gras des alcools gras, des céramides, ou des dérivés de ceux-ci, ou des séquences nucléotidiques d'adressages.

Les groupements Z peuvent également représenter des agents actifs identiques ou différents. **Il** peut s'agir par exemple d'agents thérapeutiques, d'agents de diagnostic, d'un anti-inflammatoire, d'un antimicrobien, d'un antibiotique, d'un facteur de croissance, d'une enzyme, d'un composé antioxydant, polyphénols, des tanins, des anthocyanes, des lycopènes, des terpenoides et le resvératrol.

Dans la présente, le groupement Z peut être avantageusement un acide gras saturé ou insaturé. **Il** peut s'agir par exemple d'un acide gras choisi dans le groupe comprenant l'acide acétique, l'acide caprylique, l'acide caprique, l'acide laurique, l'acide myristique, l'acide palmitique, l'acide stéarique, l'acide arachidique, l'acide béhénique, l'acide lignocérique, l'acide cérotique, l'acide myristoléique, l'acide palmitoléique, l'acide sapiénique, l'acide oléique, l'acide élaïdique, l'acide trans-vaccénique, l'acide linoléique, l'acide linolélaïdique, l'acide α-linolénique, l'acide γ-linolénique, l'acide dihomo-γ-linolénique, l'acide arachidonique, l'acide eicosapentaénoïque, l'acide clupanodonique ou l'acide docosahexaénoïque. De préférence, l'acide gras est l'acide acétique.

Dans la présente, le groupement Z peut être avantageusement un acide aminé de la série L ou D choisi dans le groupe comprenant l'alanine, l'asparagine, une chaine aromatique par exemple la tyrosine, la phénylalanine, le tryptophane, la thyroxine ou l'histidine. De préférence, l'acide aminé est la phénylalanine.

Dans la présente, le groupement Z peut être un antioxydant, par exemple la vitamine A, C, E, B9, B6, le glutathion, le sélénium, les polyphenols, par exemple les catéchines, par exemple du thé vert, les flavonoïdes, les tanins, les anthocyanes, par exemple des fruits rouges, les lycopènes, les terpenoïdes et le resvératrol.

Dans la présente, le groupement Z peut être des composés anti-âges, par exemple des rétinoïdes.

Avantageusement, les groupements Z peuvent conférer aux polymères des propriétés biologiques ou physicochimiques supplémentaires. Par exemple les groupements Z peuvent augmenter la solubilité ou la lipophilie dudit polymère permettant par exemple une meilleure diffusion ou pénétration tissulaire.

Avantageusement, les groupements Z peuvent conférer aux polymères des propriétés biologiques ou physicochimiques supplémentaires Ainsi, les polymères de l'invention, par exemple lorsque le groupement Z est choisi parmi un composé antioxydant, un composé anti-âge, les polymères de l'invention peuvent avantageusement véhiculer ces composés et ainsi fournir un effet biologique additionnel et/ou complémentaire.

Des polymères dans lesquels Z est présent répondent à la formule II suivante :

Aa Xx Yy Zz (II)

dans laquelle, A, X, Y, a, x, y sont tel que défini ci-dessus et z représente le taux de substitution par des groupements Z.

Dans la présente le taux de substitution par des groupements Z représenté par « z » peut être compris de 1 à 50%, de 10 à 25%, de préférence égale à 15, 20 ou 25%.

Les groupements X, Y et Z peuvent être indépendamment fixés sur le monomère A et/ou indépendamment fixés les uns aux autres. Lorsqu'au moins un des groupements X, Y et Z est indépendamment fixé sur un groupement X, Y et Z différent du premier, un desdits groupements X, Y ou Z est fixé au monomère A.

Ainsi, les groupements Z peuvent être fixés par covalence directement sur les monomères A ou fixés par covalence sur les groupements X et/ou Y.

Dans la présente les groupements Z peuvent aussi être conjugués aux polymères de formule AaXxYy par des liaisons autres que covalentes, par exemple par des liaisons ioniques, par exemple via des interactions ioniques, des liaisons hydrophiles ou des liaisons hydrophobes. Les polymères de l'invention peuvent alors constituer un système de vectorisation de Z.

Dans la présente, le polymère peut être par exemple un RGTA choisi dans le groupe comprenant les composés OTR4120, OTR41201, OTR41202, OTR41203, OTR41205, OTR41210 OTR41301, OTR41302, OTR41303, OTR41305, OTR 41310, OTR3131.

Dans la présente, le polymère peut être par exemple un RGTA choisi dans le groupe comprenant les composés OTR41201, OTR41202, OTR41203, OTR41205, OTR41210, OTR4120, OTR4122, OTR4125, OTR41301, OTR41302, OTR41303, OTR41305, OTR41310, OTR3131, OTR4132, OTR4135, OTR415 avec les caractéristiques mentionnées dans le tableau 1 ci-dessous

**Tableau 1 : Polymères des familles Aa Xx Yy et Aa Xx Yy Zz dans lesquels A est le glucose (PM 180D), X est CarboxyMethyl (PM 58 D) Y : SO3⁻ (PM 80D) et Z Acetate (PM 43D) ou phénylalanine (PM 165D).**

| polymère | A : glucose | | X -CH2COO | Y -SO3⁻ | Z -OCCH3 | Z phénylalanine |
|---|---|---|---|---|---|---|
| Nom du RGTA | Polymère de Dextran de départ (PM en Dalton) | Poids M moléculaire moyen +/-15% | % de substitution CM /glucose | % de substitution S0₄ / glucose) | % de substitution OCCH3 /glucose | |
| CMDS OTR41201 | 1 500 | 3 000 | 60+/-20 | 150+/-20 | 0 | |
| CMDS OTR41202 | 3 000 | 6 000 | 60+/-20 | 150+/-20 | 0 | |
| CMDS OTR41203 | 5000 | 10 000 | 60+/-20 | 150+/-20 | 0 | |
| CMDS OTR41205 | 10 000 | 20 000 | 60+/-20 | 150+/-20 | 0 | |
| CMDS OTR41210 | 20 000 | 40 000 | 60+/-20 | 150+/-20 | 0 | |
| CMDS OTR4120 | 40 000 | 80 000 | 60+/-20 | 150+/-20 | 0 | |
| CMDS OTR4122 | 110 000 | 220 000 | 60+/-20 | 150+/-20 | 0 | |
| CMDS OTR4125 | 250 000 | 500 000 | 60+/-20 | 150+/-20 | 0 | |
| CMDSA OTR41301 | 1 500 | 3 000 | 60+/-20 | 140+/-20 | 20+/-5 | |
| CMDSA OTR41302 | 3 000 | 6 000 | 60+/-20 | 140+/-20 | 20+/-5 | |
| CMDSA OTR41303 | 5000 | 10 000 | 60+/-20 | 140+/-20 | 20+/-5 | |
| CMDSA OTR4135 | 10 000 | 20 000 | 60+/-20 | 140+/-20 | 20+/-5 | |
| CMDSA OTR41310 | 20 000 | 40 000 | 60+/-20 | 140+/-20 | 20+/-5 | |
| CMDSA OTR4131 | 40 000 | 80 000 | 60+/-20 | 140+/-20 | 20+/-5 | |
| CMDSA OTR4132 | 110 000 | 220 000 | 60+/-20 | 140+/-20 | 20+/-5 | |
| CMDSA OTR4135 | 250 000 | 500 000 | 60+/-20 | 140+/-20 | 20+/-5 | |
| CMDSP OTR415 | | 5000 | 60+/-20 | 70+/-15 | - | 15+/-5 |

Dans la présente, la composition peut comprendre une concentration de 0,1 à 100µg/mL en poids de polymère biocompatible par rapport au volume de la composition. Par exemple la composition peut comprendre une concentration préférée de 1 à 10µg/mL en poids de polymère biocompatible par rapport au volume total de la composition.

Dans la présente, la composition peut être formulée et/ou adaptée selon son administration. Par exemple, pour une administration par voie topique la composition peut comprendre de 0,1 à 100 µg/mL en poids de polymère biocompatible par rapport au volume total de la composition

Par exemple, pour une administration par voie parentérale la composition peut être administrée afin de délivrer une dose de polymère biocompatible comprise de 0,1 à 5 mg par kilogramme de poids corporel.

Avantageusement, le poids moléculaire des polymères biocompatibles présents dans la composition peuvent être choisi en fonction de la voie d'administration de la composition et de la cible, par exemple épiderme, derme, muqueuses, cornée, membrane tympanique, organes, liquide intra articulaire, intraoculaire.

Par exemple, pour une administration par voie topique ou orale le poids moléculaire peut être compris de 3000 à 6000 Daltons pour favoriser le passage de membranes basales. Par exemple, pour une administration par injection par voie intradermique ou sous cutanée, ou intrathécale, ou dans les muscles ou organes ou dans les espaces intra articulaires le poids moléculaire peut être compris de 6000 à 2 500 000 daltons, de préférence de 20 000 à 250 000 daltons et par exemple de 75 000 à 150 000 daltons.

Avantageusement, lorsque le poids moléculaire de polymère biocompatible est compris de 3000 à 6000 Daltons, il peut permettre avantageusement lors de l'application topique, par exemple sur un épithélium, par exemple de muqueuses ou de la peau, un passage de la lame basale permettant avantageusement une meilleure administration et favoriser un effet sur une plus grande distance.

Avantageusement, lorsque le poids moléculaire de polymère biocompatible est compris de 3000 à 2 500 000 Daltons, il peut permettre avantageusement une meilleure disponibilité et durée de vie quelle que soit la localisation tissulaire.

Dans la présente par « acide hyaluronique» on entend tout acide hyaluronique connu de l'homme de l'Homme du métier, par exemple un glycosaminoglycane linéaire non sulfaté composé d'unités répétitives d'acide D-glucuronique et de N-acétyl-D-glucosamine. Il peut s'agir par exemple d'acide hyaluronique (HA) sous sa forme acide ou sous forme de sel (hyaluronate), d'acide hyaluronique réticulé Le HA est un glycosaminoglycane linéaire non sulfaté composé d'unités répétitives de D-acide glucuronique et de N-acetyl-D-glucosamine (Tammi R., Agren UM., Tuhkanen AL., Tammi M. Hyaluronan metabolism in skin. Progress in Histochemistry & Cytochemistry. 29(2):1-81, 1994 [15]). Il peut s'agir par exemple d'acide hyaluronique ayant des fractions de poids moléculaire moyen de 5 000 à 3 000 000 Dalton, de préférence entre 50 000 et 2 000 000 Dalton. Dans la présente l'acide hyaluronique peut être obtenu par tout procédé connu de l'homme du métier. Il peut s'agir par exemple de procédés décrits dans la revue Hyaluronan fragments: an information-rich system (R. Stern et al., European Journal of Cell Biology 58 (2006) 699-715[16]). Il peut s'agir également d'acide hyaluronique naturel ou modifiés, disponible dans le commerce, quel que soit leurs désignations et/ou poids moléculaire, par exemple d'acide hyaluronique commercial choisi parmi Hyactive CPN ; Cristalhyal ; Nutra HA ; Oligo HA; D Factor; Hyaluderm ; juvelift ; Restylane; Revitacare sans que cette liste soit exhaustive. Il peut s'agir également d'acide hyaluronique commercialisé par la société Contipro (https://www.contipro.com/portfolio/manufacturer-of-anti-ageing-cosmetic-raw-materials/HyActive) et/ou Givaudan (https://www.givaudan.com/fragrances/active-beauty/products/cristalhyal%C2%AE-range)

Dans la présente, la composition peut comprendre une concentration de 0,1 à 5% en poids d'acide hyaluronique par rapport au poids total de la composition. Par exemple la composition peut comprendre une concentration de 0,5% à 2,5% en poids d'acide hyaluronique par rapport au poids total de la composition.

Dans la présente, la composition peut être formulée et/ou adaptée selon son administration. Par exemple, pour une administration par voie topique la composition peut comprendre de 0,5% à 2,5% en poids d'acide hyaluronique par rapport au poids total de la composition

Par exemple, pour une administration par voie parentérale, par exemple intra-articulaire, par exemple dans le liquide synovial du genou ou dans le liquide synovial de l'articulation trapézo-métacarpienne ou de l'articulation métacarpophalangiène, par exemple pour une rhizartrose, ou par exemple intra-tendineuse, par exemple tendinite la composition peut être administrée afin de délivrer une dose de 1 mg à 20 mg d'acide hyaluronique par mL de composition. Par exemple pour une administration par voie parentérale la composition peut comprendre une concentration de 0,1 à 20 mg/mL d'acide hyaluronique.

Avantageusement, le poids moléculaire des polymères biocompatibles présents dans la composition peuvent être choisi en fonction de la voie d'administration de la composition.

Dans la présente, par « composition pharmaceutique » on entend toute forme de composition pharmaceutique connue de l'Homme du métier. Dans la présente, la composition pharmaceutique peut être par exemple une composition pour application topique, une solution injectable, par exemple pour une injection locale ou systémique, par exemple en sérum physiologique, en solution glucose injectable, en présence d'excipients, par exemple de Dextrans, par exemple à des concentrations connues de l'homme du métier, par exemple du microgramme à quelques milligrammes par mL.

La composition pharmaceutique peut être par exemple un médicament destiné à une administration orale choisie dans le groupe comprenant une formulation liquide, une forme posologique effervescente orale, une poudre orale, un système multiparticule, une forme galénique orodispersible.

Par exemple, lorsque la composition pharmaceutique est pour administration orale, elle peut être sous la forme d'une formulation liquide choisie dans le groupe comprenant une solution, un sirop, une suspension, une émulsion. Lorsque la composition pharmaceutique est sous la forme d'une forme posologique effervescente orale, elle peut être sous une forme choisie dans le groupe comprenant les comprimés, les granules, les poudres. Lorsque la composition pharmaceutique est sous la forme d'une poudre orale ou un système multiparticulaire, il peut être sous une forme choisie dans le groupe comprenant des billes, des granulés, des mini-comprimés et les microgranules. Lorsque la composition pharmaceutique est sous la forme d'une forme posologique orodispersible, elle peut être sous une forme choisie dans le groupe comprenant des comprimés orodispersibles, gaufrettes lyophilisées, films minces, un comprimé à mâcher, d'un comprimé, d'une capsule ou d'une gomme médicale à mâcher.

Selon la présente invention, la composition pharmaceutique peut être une composition pharmaceutique pour administration par voie orale, par exemple buccale et/ou sublingual, par exemple choisie dans le groupe comprenant les comprimés buccaux ou sublinguaux, des pates dentaires, des pansements adhésifs buccaux dentaire, les pastilles, gouttes, une solution pour pulvérisations.

Selon la présente invention, la composition pharmaceutique peut être une composition pharmaceutique pour administration par voie intra-urinaire. Avantageusement, lorsque la composition pharmaceutique est adaptée pour une administration par voie intra-urinaire elle peut attendre après administration l'épithélium de la vessie.

Selon la présente invention, la composition pharmaceutique peut être une composition dermatologique ou cosmétique, par exemple pour administration et/ou application topique.

La composition dermatologique ou cosmétique selon l'invention peut comprendre un ou plusieurs supports dematologiquement et/ou cosmétiquement acceptable(s). Dans la présente, par « support dermatologiquement et/ou cosmétiquement acceptable » on entend tout support cosmétique connu de l'homme du métier, il peut s'agir par exemple de tout support cosmétique pouvant être cité dans le dictionnaire INCI (International Nomenclature of Cosmetic Ingredients) publié par le PCPC (Personal Care Products Council).

La composition dermatologique ou cosmétique selon l'invention peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des agents épaississants minéraux, des agents épaississants organiques, associatifs ou non, des filtres solaires organiques hydrosolubles et liposolubles, des filtres solaires minéraux, des composés siliconés, des parfums, des conservateurs, des céramides, et pseudo-céramides, des vitamines et les provitamines, des protéines, des agents séquestrants, des agents alcanisants, des agents acidifiants, des agents réducteurs, des agents oxydants, des charges minérales, des colorants ou tout autre adjuvant pouvant être cité dans le dictionnaire INCI (International Nomenclature of Cosmetic Ingredients) publié par le PCPC (Personal Care Products Council).

Selon l'invention, la composition cosmétique peut, par exemple, se présenter sous toute forme connue de l'homme du métier. Il peut s'agir, par exemple d'une émulsion huile-dans-l'eau, eau-dans-l'huile, eau dans silicone, d'une émulsion multiple, d'une microémulsion, d'une nano-émulsion, d'une émulsion solide, d'un gel aqueux ou hydro-alcoolique, d'une crème, d'un gel, d'un lait, d'une lotion, d'une pommade, d'une huile, d'un baume, d'un onguent, d'un masque, d'une poudre, d'un support imbibé, par exemple un patch transdermique, un pansement imbibé, d'une lotion aqueuse, d'un spray, d'une ou hydroalcoolique et/ou une cire, d'un shampooing, d'un après-shampooing, d'un masque, sérum, lotion capillaire. De préférence, la composition cosmétique selon l'invention peut être sous une forme choisie parmi une crème, un gel, un onguent, une huile.

La composition cosmétique ou dermatologique de l'invention peut être obtenue par tout procédé approprié connu de l'homme du métier pour la fabrication d'une composition cosmétique et/ou dermatologique

Selon la présente invention, la composition pharmaceutique peut être une composition pharmaceutique pour administration par voie respiratoire ou nasale, par exemple sous la forme d'un aérosol.

Selon la présente invention, la composition peut être une composition pour nasale ou respiratoire nasale ou respiratoire, par exemple choisie dans le groupe comprenant des gouttes nasales, spray nasal, de la poudre nasale, des aérosols, par exemple des aérosols et/ou spray nasal à gaz comprimé, ou des nébuliseurs

Avantageusement, lorsque la composition pharmaceutique est adaptée pour une nasale ou par voie respiratoire, elle peut avantageusement être à visées broncho-pulmonaires.

Selon la présente invention, la composition de la présente invention peut être une composition pour administration parentérale, par exemple sous-cutanée, intramusculaire, intraveineuse, intrathécale.

Selon la présente invention, la composition peut être une composition pour administration oculaire, par exemple choisie dans le groupe comprenant des gouttes, gel, crème. Il peut s'agir par exemple de collyre, par exemple pour le traitement de la cornée, par application de la composition à la surface de l'œil, par injection trans-cornéenne par exemple dans le traitement de la membrane de Decemet, ou encore pour le traitement du Glaucome afin réduire la fibrose du canal de Schlemm par injection par exemple dans l'humeur aqueuse ou vitrée, par application topique, par exemple sur la cornée.

Avantageusement, lorsque la composition est utilisée en application topique sur la cornée, elle peut être avantageusement utile au niveau trans-cornéen, avantageusement si la composition comprend des polymères de petits poids moléculaires, par exemple compris de 2000 à 6000 Daltons.

La composition de la présente invention peut également comprendre au moins un autre ingrédient actif, particulièrement un autre ingrédient thérapeutiquement actif, par exemple pour une utilisation simultanée, séparée ou étalée dans le temps suivant la formulation galénique utilisée. Cet autre ingrédient peut être par exemple un ingrédient actif utilisé par exemple dans le traitement de maladies opportunes ou une vitamine ou un antalgique etc.

Dans la présente, l'administration du polymère biocompatible et de l'acide hyaluronique peut être simultanée, successive ou concomitante.

Selon l'invention, au moins une des administrations peut être réalisée par voie topique, orale, respiratoire ou par injection. Les deux administrations peuvent être réalisées de la même manière ou différemment. Par exemple, l'administration du polymère biocompatible et de l'acide hyaluronique peut être faite par application topique. L'administration peut être également fonction de la zone et/ou du tissu biologique à traiter.

Selon l'invention, la composition peut être, par exemple, administrée une seule fois.

Selon l'invention, la composition peut être en outre, par exemple, administrée quotidiennement, biquotidiennement et hebdomadairement ou moins Il peut s'agir par exemple d'une administration une fois par jour, deux fois par jour ou moins, par exemple une fois tous les deux jours, ou par semaine.

Selon l'invention, et le mode d'administration la composition peut être, par exemple, pour une crème administrée quotidiennement, biquotidiennement et hebdomadairement ou moins. Il peut s'agir par exemple d'une administration une fois par jour, deux fois par jour ou moins.

Selon l'invention, la composition peut être, par exemple, administrée sur une durée de 1 jour à 3 mois, par exemple pendant 2 mois. Par exemple, la composition peut être administrée sur une période de 3 ou 6 mois, par exemple avec une fréquence d'administration tous les jours.

Par exemple, lorsque la composition est sous une forme injectable, la composition peut être administrée avec une fréquence d'administration tous les 3 ou 6 mois ou moins.

L'inventeur a démontré de manière surprenante que la combinaison d'un polymère biocompatible de formule AaXxYy ou AaXxYyZz et de l'acide hyaluronique naturel ou modifié permet avantageusement et de manière surprenante d'obtenir un effet synergique dans le traitement. En particulier, l'inventeur a démontré que l'effet obtenu était à la fois une synergie allant au-delà des effets individuels de chacun des composés et également avantageusement une augmentation de la durée de ces effets.

En outre, l'inventeur a démontré de manière surprenante que l'augmentation de la durée des effets pouvait être encore augmentée, par exemple en utilisant plusieurs administrations, par exemple par des voies d'administration identique ou différente, et/ou une posologie d'administration particulière. Par exemple, l'administration de la composition selon l'invention par injection, par exemple sous-cutané, intramusculaire, peut être suivie d'une d'application de la composition selon l'invention par voie cutané, par exemple par application topique.

Dans la présente, la composition selon l'invention peut être administrée par différentes voies de manière simultanée, successive ou concomitante.

Selon l'invention, au moins une des administrations peut être réalisée par voie topique, orale ou par injection. Les deux administrations peuvent être réalisées de la même manière ou différemment. Par exemple, l'administration de la composition peut être faite par injection suivie d'une application topique de la composition. L'administration peut être également fonction de la zone et/ou du tissu biologique à traiter.

Avantageusement, lorsque l'administration de la composition peut être faite par injection suivie d'une application topique de la composition, la composition pour application topique peut être sous une forme choisie parmi une crème, gels ou sérum.

Avantageusement, l'inventeur a démontré de manière surprenant que l'application topique de la composition sous une forme choisie parmi une crème, gels ou sérum permet de manière surprenante de prolonger encore l'effet biologique dû à la première administration.

La présente invention a également pour objet une composition cosmétique ou dermatologique comprenant un polymère biocompatible de formule AaXxYy ou AaXxYyZz et de l'acide hyaluronique naturel ou modifié pour son utilisation dans le traitement cosmétique non thérapeutique du vieillissement cutané, des rides et/ou ridules, pour la protection du cuir chevelu et la régénération capillaire.

Le polymère biocompatible est tel que défini ci-dessus.

L'acide hyaluronique est tel que défini ci-dessus.

Selon l'invention, le patient peut être tout mammifère. Il peut s'agir par exemple d'un animal ou d'un être humain.

Selon l'invention, le mode et/ou la voie d'administration du polymère biocompatible peut être tel(s) que défini(s) ci-dessus.

Selon l'invention, le mode et/ou la voie d'administration de l'acide hyaluronique peut être tel(s) que défini(s) ci-dessus, de préférence par injection dans le tissu biologique.

Selon l'invention la fréquence d'administration du polymère biocompatible peut être telle que définie ci-dessus.

Selon l'invention la fréquence d'administration de l'acide hyaluronique peut être telle que définie ci-dessus.

La présente invention a également pour objet une composition cosmétique ou dermatologique comprenant un polymère biocompatible de formule AaXxYy ou AaXxYyZz et de l'acide hyaluronique naturel ou modifié pour son utilisation pour la prévention et/ou le traitement cosmétique non thérapeutique de la chute des cheveux et/ou de l'alopécie.

Le polymère biocompatible est tel que défini ci-dessus.

L'acide hyaluronique est tel que défini ci-dessus.

Selon l'invention, le mode et/ou la voie d'administration du polymère biocompatible peut être tel(s) que défini(s) ci-dessus.

Selon l'invention, le mode et/ou la voie d'administration de l'acide hyaluronique peut être tel(s) que défini(s) ci-dessus, de préférence par injection dans le tissu biologique.

Selon l'invention, la composition cosmétique ou dermatologique comprenant un polymère biocompatible de formule AaXxYy ou AaXxYyZz et de l'acide hyaluronique peut être une composition pour application topique et/ou capillaire, par exemple une crème.

Selon l'invention la fréquence d'administration du polymère biocompatible peut être telle que définie ci-dessus.

Selon l'invention la fréquence d'administration de l'acide hyaluronique peut être telle que définie ci-dessus.

La présente invention a également pour objet une composition cosmétique ou dermatologique anti-âge et/ou pour la protection de la peau des agressions extérieures et/ou pour le traitement et/ou la prévention du vieillissement de la peau comprenant un polymère biocompatible de formule AaXxYy ou AaXxYyZz et de l'acide hyaluronique.

Le polymère biocompatible est tel que défini ci-dessus.

L'acide hyaluronique est tel que défini ci-dessus.

Selon l'invention, le patient peut être tout mammifère. Il peut s'agir par exemple d'un animal ou d'un être humain.

Selon l'invention, le mode et/ou la voie d'administration du polymère biocompatible peut être tel(s) que défini(s) ci-dessus.

Selon l'invention, le mode et/ou la voie d'administration de l'acide hyaluronique peut être tel(s) que défini(s) ci-dessus, de préférence par injection dans le tissu biologique.

Selon l'invention la fréquence d'administration du polymère biocompatible peut être telle que définie ci-dessus.

Selon l'invention la fréquence d'administration de l'acide hyaluronique peut être telle que définie ci-dessus.

La présente invention a également pour objet un procédé de traitement cosmétique comprenant l'application sur la peau d'une composition cosmétique comprenant un polymère biocompatible de formule AaXxYy ou AaXxYyZz et de l'acide hyaluronique.

Le polymère biocompatible est tel que défini ci-dessus.

L'acide hyaluronique est tel que défini ci-dessus.

Selon l'invention, le patient peut être tout mammifère. Il peut s'agir par exemple d'un animal ou d'un être humain.

Selon l'invention, le mode et/ou la voie d'administration du polymère biocompatible peut être tel(s) que défini(s) ci-dessus.

Selon l'invention, le mode et/ou la voie d'administration de l'acide hyaluronique peut être tel(s) que défini(s) ci-dessus, de préférence par injection dans le tissu biologique.

Selon l'invention la fréquence d'administration du polymère biocompatible peut être telle que définie ci-dessus.

Selon l'invention la fréquence d'administration de l'acide hyaluronique peut être telle que définie ci-dessus.

Dans la présente par traitement cosmétique on entend un traitement cosmétique non thérapeutique.

Selon l'invention, le traitement cosmétique non thérapeutique peut-être un traitement cosmétique anti-âge, un traitement cosmétique pour la prévention du vieillissement cutané, un traitement cosmétique du vieillissement cutané et/ou un traitement cosmétique des peaux matures.

En effet, tel que mentionné ci-dessus, les inventeurs de la présente invention ont démontré de manière surprenante que la composition selon l'invention permet de stimuler de manière synergique l'hydratation des tissus, avantageusement sur la durée et lié notamment à la création d'un meilleur environnement tissulaire prolongeant de manière surprenante l'effet de l'acide hyaluronique, tout en réduisant l'activité des glycanases et en stimulant la réponse des cellules proximales, permettant avantageusement et de manière surprenante une néo-synthèse des constituants de la matrice extracellulaire, ces constituants étant de meilleure qualité, tout en restaurant la distribution et la polarité de la matrice.

Pour cette application, on peut, par exemple, utiliser les formes galéniques décrites ci-dessus. L'application sur la peau peut donc être réalisée en fonction de la forme galénique utilisée.

Il peut s'agir, par exemple d'une simple application sur la peau ou d'une application accompagnée d'un massage de la peau avec la composition de la présente invention.

Il peut s'agir, par exemple d'une application successive de la composition selon l'invention comprenant une première application, par exemple par injection cutanée, de la composition selon l'invention, suivi d'une application topique de ladite composition selon l'invention.

L'application est de préférence réalisée avec une quantité suffisante de la composition, par exemple afin que toute la surface de la peau à traiter soit traitée. Il peut s'agir par exemple d'une application classique, comme une crème sur la peau. Il peut s'agir par exemple aussi d'une application pour former un masque traitant.

L'application peut être, par exemple une application quotidienne, hebdomadaire et bi-mensuelle. Il peut s'agir par exemple d'une application une fois par jour, deux fois par jour ou moins.

L'inventeur a également démontré de manière surprenante que la composition selon l'invention permet avantageusement de combler les rides avec une efficacité accrue, tout en évitant d'éventuels effets secondaires par rapport au produit connu, par exemple le collagène et/ou les injections de toxine botulique. En outre, l'inventeur a démontré que la composition selon l'invention peut être utile comme agent de comblement, par exemple de rides, mais également pour injection dans des traitements esthétiques et/ou comme agent de remplissage, par exemple pour l'hydratation et de gonflement, par exemple au niveau des organes génitaux et/ou sexuel des hommes ou des femmes.

La présente invention a donc également pour objet l'utilisation d'une composition comprenant un polymère biocompatible de formule AaXxYy ou AaXxYyZz et de l'acide hyaluronique selon l'invention comme agent de comblement et/ou remplissage, par exemple d'organes et/ou de tissus biologique.

La présente invention a donc également pour objet l'utilisation cosmétique d'une composition comprenant un polymère biocompatible de formule AaXxYy ou AaXxYyZz et de l'acide hyaluronique selon l'invention comme agent de comblement et/ou remplissage de la peau.

L'inventeur a également démontré de manière surprenante que la composition selon l'invention permet avantageusement d'améliorer la cicatrisation de lésions tissues biologiques, en particulier de part une favorisation de l'hydratation du tissu et du comblement de la lésion. En particulier, l'inventeur a démontré de manière surprenant que la composition selon l'invention permet avantageusement de favoriser la cicatrisation cutanée et/ou d'améliorer l'aspect de lésions cutanées, par exemple de cicatrice.

La présente invention a donc également pour objet l'utilisation cosmétique d'une composition comprenant un polymère biocompatible de formule AaXxYy ou AaXxYyZz et de l'acide hyaluronique selon l'invention pour l'amélioration de l'aspect de la peau, par exemple l'améliorer l'aspect de lésions cutanés, par exemple de cicatrice.

La présente invention a donc également pour objet l'utilisation cosmétique d'une composition comprenant un polymère biocompatible de formule AaXxYy ou AaXxYyZz et de l'acide hyaluronique selon l'invention pour le traitement de lésions cutanées.

L'inventeur a également démontré de manière surprenante que la composition selon l'invention permet avantageusement d'améliorer la cicatrisation de lésions de tissues biologiques, en particulier de part une favorisation de l'hydratation du tissu, la prévention de la formation de fibrose et du comblement de la lésion.

En particulier, l'inventeur a démontré que la composition selon l'invention permet avantageusement d'accélérer la vitesse et la qualité physique du tissu cicatriciel, en particulier ces propriétés mécaniques, sa souplesse et son hydratation.

En outre, l'inventeur a démontré, notamment dans les exemples, que les lésions susceptibles d'être traitées par la composition selon l'invention peuvent être à tous types de lésions tissulaires quel qu'en soient l'origine ainsi qu'à tout type de tissu ou organe. En particulier, l'homme du métier, à la lumière des exemples ci-dessous dans lesquels une grande diversité de lésions sont effectivement traitées, comprend aisément et peut, à la lumière de ces connaissances extrapoler les autres lésions tissulaires susceptibles d'être traitées par la présente invention.

Dans la présente par « lésions tissulaires » on entend toute lésion de tout tissu biologique d'un mammifère connu de l'homme du métier. Il peut s'agir par exemple d'une lésion du tissu conjonctif, du tissu musculaire, du tissu osseux, cartilagineux et/ou du tissu épithéliale. Il peut s'agir par exemple de toute lésion de tout organe, organelle de mammifère connu de l'homme du métier. Il peut s'agir par exemple d'une lésion de tissus du tractus digestif, de tissus du tractus gastro-intestinal, du système digestif alimentation et excrétion, du tractus génital, du système reproducteur, du système optique, olfactif ou auditif, du système sensoriel, du système circulatoire et/ou cardiovasculaire, du système respiratoire, du système musculaire, du système locomoteur. Il peut s'agir par exemple d'une lésion du tissu gastrique, d'une lésion buccale, d'une lésion de la cornée, d'une lésion tympanique, d'une lésion de la cochlée, d'une lésion de la peau, par exemple une plaie, une plaie chronique, par exemple une plaie du diabétique, une plaie ulcéreuse, un escarre, une brulure cutanée, une plaie nécrosante, une lésion veineuse, une lésion ischémique, par exemple une nécrose ischémique, une lésion due à un infarctus, par exemple une infarctus du myocarde, une lésion osseuse, par exemple une fracture, une fracture avec défaut osseux, une ostéonécrose (« non union bone fracture »), une lésion ostéochondrale, une lésion cartilagineuse, une lésion tendineuse, une lésion chirurgicale, une lésion due à une opération chirurgicale, une lésion due à un traitement médicale, par exemple une radiothérapie, une lésion du tissu nerveux, par exemple une lésion du cerveau, par exemple une lésion due à une exérèse d'une tumeur, une lésion de la moelle épinière, une lésion de fibre nerveuse, par exemple du système locomoteur et/ou sensoriel, une lésion du système respiratoire, par exemple des lésions pulmonaires, une lésion du système circulatoire, par exemple une lésion des artères et/ou des vaisseaux, une lésion du système digestif, hépatique, rénal, urinaire

La présente invention a également pour objet une composition comprenant un polymère biocompatible de formule AaXxYy ou AaXxYyZz et de l'acide hyaluronique selon l'invention pour son utilisation comme médicament..

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de lésions tissulaires.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de lésions tissulaires choisi parmi les lésions oculaires, les lésions des cordes vocales, les lésions articulaires, les lésions discales les lésions tendineuses et/ou les lésions ligamentaire, les lésions de la rétine, par exemple un décollement de la rétine, les lésions dues à la maladie de Behçet, les lésions de surface, par exemple les plaies cutanées, les ulcères, par exemple les ulcères de plaies diabétique, de l'estomac.

L'inventeur a également démontré que la composition selon l'invention permet avantageusement après administration dans une articulation peut être un facilitateur/hydratant des articulations et/ou un lubrifiant de l'articulation permettant avantageusement de prévenir et/ou traiter des inflammations articulaires, de prévenir et/ou traiter l'arthrite et/ou l'arthrose.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de pathologies et/ou conditions du système locomoteur.

Selon l'invention par pathologie et/ou conditions du système locomoteur on entend toute pathologie et/ou conditions connues de l'homme du métier susceptible de modifier et/ou altérer le fonctionnement du système locomoteur. Il peut s'agir par exemple d'une pathologie articulaire, par exemple une inflammation articulaire, de l'arthrose de l'arthrite. Il peut s'agir par exemple d'une pathologie au niveau des tendons ou des ligaments, par exemple une inflammation, un étirement, un aplatissement, une lésion, une rupture ou arrachement. Il peut s'agir par exemple d'une pathologie au niveau de la colonne vertébrale et /ou des discs intervertébraux, par exemple d'un déplacement, aplatissement ou tassement ou écrasement lié à des traumas, à l'âge, à la déshydratation etc.. ;

L'inventeur a également démontré que la composition selon l'invention permet avantageusement après administration dans l'œil un remplissage et renforcement de l'environnement sub-rétinien notamment dans détachements rétiniens et avantageusement une protection du microenvironnement rétinien dans les DMLA sèches et humide permettant avantageusement de prévenir et/ou traiter les lésions de la rétine, par exemple un décollement de la rétine, et/ou le traitement de la .Dégénérescence Maculaire Liée à l'Age, la Dégénérescence Maculaire Liée à l'Age sèche et la Dégénérescence Maculaire Liée à l'Age humide

L'inventeur a également démontré de manière surprenante que la composition selon l'invention permet avantageusement d'améliorer l'hydratation de tissu.

En outre, l'inventeur a démontré, notamment dans les exemples, que la composition selon l'invention permet de traiter et/ou améliorer l'hydratation de tous types de tissu ou organe. En particulier, l'homme du métier, à la lumière des exemples ci-dessous dans lesquels une grande diversité de tissus et organes sont effectivement traitées, comprend aisément et peut, à la lumière de ces connaissances extrapoler tissus et organes susceptibles d'être traitées par la présente invention.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de la sécheresse tissulaire.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de la sécheresse tissulaires choisi notamment parmi la sécheresse des muqueuses naturellement humides par exemple la surface oculaire, les muqueuses buccales, vaginales, nasales, les tissus en contact avec les fluides biologiques comme liquides synoviaux, cephalospinal, péritonéal, péricardiaque, la moelle osseuse, les tissus articulaires, les épithéliums par exemple de la vessie, du système lymphatique, de la moelle épinière.

En outre, l'inventeur a démontré, notamment dans les exemples, que la composition selon l'invention permet de traiter et/ou améliorer l'hydratation de muqueuse de tissu ou organe. En particulier, l'homme du métier, à la lumière des exemples ci-dessous dans lesquels une grande diversité de tissus et organes sont effectivement traitées, comprend aisément et peut, à la lumière de ces connaissances extrapoler tissus et organes susceptibles d'être traitées par la présente invention.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de la sécheresse de muqueuses, par exemple de la muqueuse vaginale, de la muqueuse buccale.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de la sécheresse oculaire.

L'inventeur a également démontré de manière surprenante que la composition selon l'invention permet avantageusement de traiter des fibroses de tissues biologiques, en particulier l'inventeur a démontré que la composition selon l'invention permet de manière surprenante une réduction de la fibrose, en particulier une diminution synergique du rapport des synthèses de collagène 3 et de collagène 1 (COL3/COL1), la fibrose étant caractérisée entre autre par une surexpression du COL 3 par rapport au COL1).

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de la fibrose.

En outre, l'inventeur a démontré, notamment dans les exemples, que les fibroses susceptibles d'être traitées par la composition selon l'invention peuvent être tous types de fibroses quel qu'en soient l'origine ainsi qu'à tout type de tissu ou organe. En particulier, l'homme du métier, à la lumière des exemples ci-dessous dans lesquels une grande diversité de fibroses sont effectivement traitées, comprend aisément et peut, à la lumière de ces connaissances extrapoler les autres fibroses susceptibles d'être traitées par la présente invention. Il peut s'agir par exemple de fibroses induites par des lésions d'ischémiques, par exemple des lésions ischémiques musculaires et/ou cardiaques. Il peut s'agir par exemple de fibroses cicatriciels après ischémie ou écrasement du muscle squelettiques, ischémie muscle cardiaque infarci, de fibroses des tissus tendineux ou ligaments, du tissus osseux par exemple un cale osseux, de fibrose du tissus digestifs, par exemple dans la maladie de Crohn, de fibroses post radiques, de fibroses hépatiques, de fibroses pulmonaires, de fibroses dues à la maladie de la Peyronie, de fibroses post chirurgie, par exemple de fibrose induisant des adhésions d'organes ou de tissus, par exemple de fibrose post chirurgie digestive, chirurgie des tendons, des nerfs, des vaisseaux, de fibroses naturelles, par exemple survenant lors d'un glaucome, par exemple une fibrose du canal de Shlemm, par exemple de fibroses survenant lors de la maladie de Dupuytren, de fibroses issus du syndrome carpiens et/ou d'autre fibroses tendineuses.

Selon l'invention, l'acide hyaluronique est tel que défini ci-dessus.

Selon l'invention, le polymère biocompatible de formule AaXxYy ou AaXxYyZz est tel que défini ci-dessus.

Selon l'invention, la composition pharmaceutique ou dermatologique est telle que définie ci-dessus.

Selon l'invention la fréquence d'administration du polymère biocompatible peut être telle que définie ci-dessus.

Selon l'invention, le mode et/ou la voie d'administration du polymère biocompatible peut être tel(s) que défini(s) ci-dessus.

L'inventeur a également démontré de manière surprenante que la composition selon l'invention peut être avantageusement utile dans le traitement de l'épidermolyse bulleuse. En particulier, les inventeurs ont démontré de manière surprenante et inattendue que la composition comprenant un polymère biocompatible de formule AaXxYy ou AaXxYyZz et de l'acide hyaluronique permet avantageusement de favoriser la cicatrisation d'ulcères chez des enfants atteints d'épidermolyse bulleuse.

L'inventeur a également démontré de manière surprenante et inattendue que la composition selon l'invention permet avantageusement de manière synergique de favoriser la cicatrisation des ulcères et également de diminuer la douleur liée à ces ulcères.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de l'épidermolyse bulleuse

Selon l'invention, l'acide hyaluronique est tel que défini ci-dessus.

Selon l'invention, le polymère biocompatible de formule AaXxYy ou AaXxYyZz est tel que défini ci-dessus.

Selon l'invention, la composition pharmaceutique ou dermatologique est telle que définie ci-dessus. Avantageusement, la composition peut être sous une forme adaptée pour une aspersion, par exemple avec un spray.

Selon l'invention la fréquence d'administration du polymère biocompatible peut être telle que définie ci-dessus.

Selon l'invention, le mode et/ou la voie d'administration du polymère biocompatible peut être tel(s) que défini(s) ci-dessus. Il peut s'agir par exemple d'une application cutanée, par exemple par aspersion, par exemple avec un pulvérisateur comprenant la composition.

L'inventeur a également démontré de manière surprenante que la composition selon l'invention peut être avantageusement utile dans le traitement de maladies parodontales.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement le traitement de maladies parodontales.

Dans la présente par maladies parodontales on entend toute maladie parodontale connue de l'homme du métier. Il peut s'agir par exemple d'une gingivite, d'une parodontite.

L'inventeur a également démontré de manière surprenante que la composition selon l'invention peut être avantageusement utile dans le traitement de maladie de la muqueuse buccale.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de pathologies de la muqueuse buccale.

Dans la présente, par pathologies de la muqueuse buccale on entend toute pathologie de la muqueuse buccale connue de l'homme du métier. Il peut s'agir par exemple d'aphtes, de gingivites, de mucites.

L'inventeur a également démontré de manière surprenante que la composition selon l'invention peut être avantageusement utile dans le traitement de brulures cutanées quelle qu'en soit la cause. Il peut s'agir par exemple de brulures thermiques, de brulures post radique, de brulures cutanées dues à l'exposition aux rayons UV, de brulures cutanées dues à l'exposition à des rayonnements ionisants, par exemple une exposition aux rayons X, aux rayons gamma.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de brulures cutanées.

Selon l'invention, l'acide hyaluronique est tel que défini ci-dessus.

Selon l'invention, le polymère biocompatible de formule AaXxYy ou AaXxYyZz est tel que défini ci-dessus. Avantageusement, le polymère biocompatible de formule AaXxYy ou AaXxYyZz peut avoir un poids moléculaire compris de 3000 à 150 000 Daltons

Selon l'invention, la composition pharmaceutique ou dermatologique est telle que définie ci-dessus. Avantageusement, la composition peut être sous une forme adaptée pour une aspersion, par exemple avec un spray et/ou un gel. Avantageusement, la composition peut être sous une forme adaptée pour une aspersion, par exemple un spray ou un gel, et ne contient pas d'acides gras.

Avantageusement, lorsque la composition est une forme adaptée pour une aspersion, par exemple un spray et/ou un gel et ne contient pas d'acides gras, elle permet avantageusement de traiter les brulures tout en évitant la présence de résidus d'acide gras évitant ainsi tout possible effet indésirable.

L'inventeur a également démontré de manière surprenante que la composition selon l'invention peut être avantageusement utile dans le traitement de maladies dégénératives.

La présente invention a donc également pour objet une composition comprenant un polymère biocompatible de formule AaXxYy ou AaXxYyZz et de l'acide hyaluronique selon l'invention pour son utilisation comme médicament pour la prévention et/ou le traitement de maladies dégénératives.

Dans la présente, par de maladies dégénératives on entend toute maladie dégénérative connue de l'homme du métier. Il peut s'agir par exemple des séquelles, par exemple liées à un accident vasculaire cérébrales, des maladies neurodégénératives, par exemple la maladie d'Alzheimer, par exemple la maladie de Parkinson, la dégénérescence de la rétine, de maladie dégénérative liées une dégradation des tissus, par exemple articulaire et/ou musculaire, des myopathies, de maladie dégénérative liées à l'âge.

Avantageusement, l'inventeur a démontré que la composition comprenant un polymère biocompatible de formule AaXxYy ou AaXxYyZz et de l'acide hyaluronique permet de recréer un meilleur microenvironnement cellulaire favorable à la survie et à la récupération fonctionnelles des tissus, permettant avantageusement un ralentissement et/ou un traitement des maladies dégénérative.

La présente invention a également pour objet une composition pour son application comme médicament ladite composition comprenant un polymère biocompatible de formule AaXxYy ou AaXxYyZz et de l'acide hyaluronique.

Selon l'invention, l'acide hyaluronique est tel que défini ci-dessus. Selon l'invention, le polymère biocompatible de formule AaXxYy ou AaXxYyZz est tel que défini ci-dessus.

La composition est telle que définie ci-dessus.

Selon l'invention la fréquence d'administration du polymère biocompatible peut être telle que définie ci-dessus.

Selon l'invention, le mode et/ou la voie d'administration du polymère biocompatible peut être tel(s) que défini(s) ci-dessus.

La présente invention se rapporte également à un procédé de traitement d'un patient comprenant dans un ordre quelconque les étapes suivantes:
i. l'administration d'au moins un polymère biocompatible, et
ii. l'administration d'acide hyaluronique,
dans lequel, les administrations sont concomitantes, successives ou alternatives.

Le polymère biocompatible est tel que défini ci-dessus.

L'acide hyaluronique est tel que défini ci-dessus.

Selon l'invention, le patient peut être tout mammifère. Il peut s'agir par exemple d'un animal ou d'un être humain.

Selon l'invention, le mode et/ou la voie d'administration du polymère biocompatible peut être tel(s) que défini(s) ci-dessus.

Selon l'invention, le mode et/ou la voie d'administration de l'acide hyaluronique peut être tel(s) que défini(s) ci-dessus, de préférence par injection dans le tissu biologique.

Selon l'invention la fréquence d'administration du polymère biocompatible peut être telle que définie ci-dessus.

Selon l'invention la fréquence d'administration de l'acide hyaluronique peut être telle que définie ci-dessus.

Selon l'invention, l'administration de d'acide hyaluronique et de polymères biocompatibles peut être simultanée, par exemple dans un seul mélange ou composition, ou successives

Selon l'invention, lorsque l'administration d'acide hyaluronique et de polymères biocompatibles sont successives, la posologie peut être, par exemple pour chaque administration, une l'administration d'acide hyaluronique suivie d'une administration de polymères biocompatibles. Par exemple, l'acide hyaluronique peut être administrées de 1 minute à 48 heures avant l'administration des polymères biocompatibles.

En d'autres termes, même si dans la présente description il est fait référence à une composition, on comprend bien que chacun des composés de la composition peut être administré concomitamment aux autres composés (par exemple dans une seule composition ou dans deux compositions, chacune de ces compositions comprenant un ou plusieurs des composants précités, le mode d'administration de chacun des composés ou composition(s) pouvant être identique ou différent), ou indépendamment les uns des autres, par exemple successivement, par exemple administration indépendante d'un polymère biocompatible et, administration indépendante d'acide hyaluronique, ces administrations étant réalisées sur un même patient, concomitamment ou successivement ou alternativement, dans un ordre qui est celui précité ou un autre ordre. Ces différentes administrations peuvent être réalisées, indépendamment l'une de l'autre ou de manière liée (composition ou co-administration), par un mode d'administration identique ou différent (injection, ingestion, application topique, etc.), une ou plusieurs fois par semaine, pendant une ou plusieurs semaines successives ou non.

Avantageusement, l'invention fournie donc une réponse générale et simple à un problème technique complexe et pour lequel un besoin réel et persistant existe dans l'état de la technique. Elle est notamment illustrée de manière non limitative dans les exemples ci-dessous facilement extrapolable pour l'homme de l'art à tous types conditions quel qu'en soient l'origine ainsi qu'à tout type de tissu ou organe.

En particulier, l'inventeur a démontré que la présente invention peut être généralisée à l'ensemble des lésions et/ou de conditions notamment de part certaines caractéristiques communes des lésions et/ou des conditions comprenant une altération de la matrice extracellulaire.

La présente invention a également pour objet un procédé ex-vivo de préparation de greffe comprenant l'imprégnation d'un greffon et/ou organe à greffer dans composition comprenant polymère biocompatible de formule AaXxYy ou AaXxYyZz et de l'acide hyaluronique telle que définie ci-dessus.

La présente invention a également pour l'utilisation composition comprenant polymère biocompatible de formule AaXxYy ou AaXxYyZz et de l'acide hyaluronique telle que définie ci-dessus pour la préparation ex-vivo d'un greffon et/ou organe.

Le polymère biocompatible est tel que défini ci-dessus.

L'acide hyaluronique est tel que défini ci-dessus.

Selon l'invention, l'imprégnation peut être réalisée par tout procédé connu de l'homme du métier. Il peut s'agir par exemple de plonger l'organe et/ou le plongeant dans une composition comprenant polymère biocompatible de formule AaXxYy ou AaXxYyZz et de l'acide hyaluronique telle que définie ci-dessus, soit par perfusion d'une composition comprenant polymère biocompatible de formule AaXxYy ou AaXxYyZz et de l'acide hyaluronique telle que définie ci-dessus, soit par aspersion d'une composition comprenant polymère biocompatible de formule AaXxYy ou AaXxYyZz et de l'acide hyaluronique telle que définie ci-dessus.

La présente invention a également pour objet l'utilisation d'une composition comprenant polymère biocompatible de formule AaXxYy ou AaXxYyZz et de l'acide hyaluronique telle que définie ci-dessus pour la préparation in-vitro et/ou ex-vivo d'un biomatériau implantable.

Par exemple pour des biomatériaux implantables, le polymère biocompatible peut être ajouté par exemple par imprégnation après fabrication du biomatériau, par exemple pour un tissu ou un organe. Il peut être par exemple aussi ajouté lors de la fabrication du biomatériau dès le départ, par exemple le polymère biocompatible de formule AaXxYy ou AaXxYyZz et d'acide hyaluronique tel que défini ci-dessous peut être ajouté par couches successives par exemple de manière similaire a des impressions 3D.

Dans la présente, par « biomatériaux implantables » on entend tout biomatériaux implantables connus de l'homme du métier et/ou disponible dans le commerce. Il peut s'agir par exemple d'un matériau implantable compatible, par exemple de toute nature, biodégradable, réticulés ou non, colonisable de préférence. Il peut s'agir de biomatériaux implantables à base de réticulation de protéines, par exemple de collagènes, de fibrine, de polysaccharides par exemple le dextran, la chitine, l'acide hyaluronique, l'alginate, la cellulose et leurs dérivatifs, de copolymères biodégradables et biocompatibles à base d'acide glycolique, lactique, malique, de polymères, par exemple pouvant prendre des transitions liquides gel par des polymérisation contrôlable par la température, ou par des enzymes ou des irradiations ou autres procédés. Il peut s'agir par exemple de polymère à base de polycaprolactone, polyurétane, polytétrafluoroethylène silicone, à base de sels inorganiques par exemple les phosphates de calcium ou les hydroxyapatites. Il peut s'agir par exemple de matériaux à base ou en céramique, en métal, par exemple en aluminium, en acier, en titane et/ou des alliages de ceux-ci. Avantageusement lorsque le matériau est un matériau à base ou en céramique et/ou en métal, l'imprégnation permet recouvrement de la surface extérieure du matériau, l'imprégnation peut être avantageusement réalisée par spray.

Selon l'invention, la composition d'imprégnation peut comprendre une concentration 0,1µg/mL à 1mg/mL de polymère biocompatible tel que défini ci-dessus.

Selon l'invention, la composition d'imprégnation peut comprendre une concentration de 0,5 mg.mL⁻¹ à 20 mg.mL⁻¹ d'acide hyaluronique tel que défini ci-dessus.

Selon l'invention, la durée d'imprégnation peut être comprise de 5 minutes à 24 heures. Avantageusement, la durée d'imprégnation peut être fonction de la structure du greffon et/ou organe et/ou du matériau implantables.

Avantageusement, l'imprégnation permet en outre d'améliorer l'efficacité de prise de greffe. En effet, l'inventeur a montré de manière surprenante que la présence du polymère et de l'acide hyaluronique dans la solution de conservation du greffon et/ou de l'organe permet avantageusement un effet synergique protecteur et anti apoptotique.

Avantageusement l'inventeur a démontré de manière surprenante que la composition selon l'invention permet l'obtention d'un effet synergique notamment sur la durée de la combinaison des propriétés régénératrice et protectrice de polymères biocompatibles de formule générale AaXxYy ou AaXxYyZz, aussi désigné RGTA et des propriétés hydratantes et mécaniques de l'acide hyaluronique natif ou modifié.

En outre l'inventeur a démontré que la composition selon l'invention permet avantageusement de prévenir et/ou traiter les défauts et/ou altérations liées à des séquelles et stress de toutes natures (mécaniques, oxydatives, irradiations ....) et au vieillissement des tissus et organes de toutes origines in vitro, ex vivo et in vivo chez l'homme et l'animal.

Tel que démontré, la composition selon l'invention peut être utilisée pour la prévention, le ralentissement et l'amélioration des marques liées au vieillissement des tissus notamment comme celles sur la peau par exemple la prévention, le ralentissement et l'amélioration de rides, de cernes, la perte d'élasticité et de souplesse ou de tension, par la présence de marques, la sécheresse cutanée, l'épaisseur de la peau et/ou la pigmentation.

Tel que démontré, la composition selon l'invention peut être utilisée pour la prévention, le ralentissement et/ou le traitement de pathologies articulaire, par exemple la détérioration des tissus cartilagineux ou -synoviaux.

Tel que démontré, la composition selon l'invention peut être utile pour la prévention, le ralentissement et/ou le traitement de l'altération de la qualité de l'épithélium et/ou du stroma cornéen et/ou de l'humeur vitrée.

Tel que démontré, la composition selon l'invention peut être utile pour la prévention, le ralentissement et/ou le traitement de l'altération de la qualité de l'épithélium et/ou du stroma vésical

Tel que démontré, la composition selon l'invention peut être utile pour la prévention, le ralentissement et/ou le traitement de tout tissus et/ou liquides biologiques dans lesquels l'acide hyaluronique est naturellement présent et/ou dont la production par les cellules et/ou tissus sous-jacents peuvent être altéré et/ou la qualité et/ou stabilité de l'acide hyaluronique peut être modifié et/ou altéré.

Tel que démontré la composition selon l'invention permet avantageusement et de manière surprenante une récupération synergique de la qualité esthétique et fonctionnelle de la peau dans son ensemble mais aussi avec d'autres tissus et se traduit par un effet de rajeunissement et de récupération fonctionnelle qu'individuellement chacun des produits seuls ne permettait pas d'obtenir, et également un effet durable inattendu.

En outre tel que démontré, une prolongation des effets obtenus par une première administration de la composition selon l'invention peut être encore améliorée par exemple un apport itéré, par exemple par voie topique, sur des épithélium/épiderme/muqueuses.

La présente invention a également pour objet les modes de réalisation suivant :
i. Composition pharmaceutique ou dermatologique pour son utilisation comme médicament, ladite composition comprenant
   - un polymère biocompatible de formule générale (I) suivante

      AaXxYy (I)

      dans laquelle :
      A représente un monomère,
      X représente un groupement R₁COOR₂ ou -R₉(C=O)R₁₀,
      Y représente un groupement O ou N-sulfonate et répondant à l'une des formules suivante -R₃OSO₃R₄, -R₅NSO₃R₆, R₇SO₃R₈ dans lesquelles :
         R₁, R₃, R₅ et R₉ représentent indépendamment une chaîne hydrocarbonée aliphatique, éventuellement ramifiée et/ou insaturée et qui contient éventuellement un ou plusieurs cycles aromatiques à l'exception de la benzylamine et de la benzylamine sulfonate, R₂, R₄, R₆ et R₈ représentent indépendamment un atome d'hydrogène ou un cation,
         R₇ et R₁₀ représente indépendamment une liaison, une chaîne hydrocarbonée aliphatique, éventuellement ramifiée et/ou insaturée,
         a représente le nombre de monomères,
         x représente le taux de substitution des monomères A par des groupements X,
         y représente le taux de substitution des monomères A par des groupements Y, et
   - De l'acide hyaluronique.
ii. Utilisation cosmétique non thérapeutique ou dermatologique d'une composition comprenant
   - un polymère biocompatible de formule générale (I) suivante

      AaXxYy (I)

      dans laquelle :
      A représente un monomère,
      X représente un groupement R₁COOR₂ ou -R₉(C=O)R₁₀,
      Y représente un groupement O ou N-sulfonate et répondant à l'une des formules suivante -R₃OSO₃R₄, -R₅NSO₃R₆, R₇SO₃R₈ dans lesquelles :
         R₁, R₃, R₅ et R₉ représentent indépendamment une chaîne hydrocarbonée aliphatique, éventuellement ramifiée et/ou insaturée et qui contient éventuellement un ou plusieurs cycles aromatiques à l'exception de la benzylamine et de la benzylamine sulfonate, R₂, R₄, R₆ et R₈ représentent indépendamment un atome d'hydrogène ou un cation,
         R₇ et R₁₀ représente indépendamment une liaison, une chaîne hydrocarbonée aliphatique, éventuellement ramifiée et/ou insaturée,
         a représente le nombre de monomères,
         x représente le taux de substitution des monomères A par des groupements X,
         y représente le taux de substitution des monomères A par des groupements Y, et
   - de l'acide hyaluronique
iii. Composition pour son application ou utilisation selon i ou ii , dans laquelle les monomères A identiques ou différents sont choisis parmi les sucres, les esters, les alcools, les acides aminés, les nucléotides, les acides nucléiques, les protéines ou des dérivés de ceux-ci.
iv. Composition pour son application ou utilisation selon l'un quelconque des i à iii, dans laquelle les monomères A identiques ou différents sont choisis parmi les sucres ou dérivés de ceux-ci.
v. Composition pour son application ou utilisation selon l'une quelconque des i à iv, dans laquelle un maximum de 20% des monomères A sont indépendamment des monomères de formule suivante : 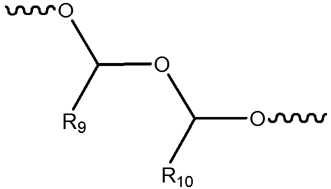 dans laquelle R₉ et R₁₀ représente indépendamment un atome d'oxygène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué, un groupement de formule -R₁₁-(X)ₙ-R₁₂ dans laquelle R₁₁ représente une chaine carbonée aliphatique en C₁ à C₄, éventuellement ramifiée et/ou insaturée, X représente un hétéroatome choisi parmi l'oxygène et l'azote, n est un entier compris de 1 à 4 et R₁₂ est un atome d'hydrogène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué.
vi. Composition pour son application ou utilisation selon l'une quelconque des i à v, dans laquelle le nombre de monomère « a » est tel que la masse desdits polymères de formule (I) est supérieure ou égale à 3000 daltons.
vii. Composition pour son application ou utilisation selon l'une quelconque des i à vi, dans laquelle le taux de substitution « x » est compris entre 10 et 150%.
viii. Composition pour son application ou utilisation selon l'un quelconque des i à vii, dans laquelle le taux de substitution « y » est compris entre 10 et 170%.
ix. Composition pour son application selon l'un quelconque des i à viii, dans laquelle que ledit polymère biocompatible comprend en outre des groupements chimiques fonctionnels Z, différents de X et Y, capables de conférer audit polymère des propriétés biologiques ou physicochimiques supplémentaires.
x. Composition pour son application selon ix, dans laquelle le taux de substitution « z » de l'ensemble des monomères A par des groupements Z est compris de 1 à 50%.
xi. Composition pour son application selon ix ou x, dans laquelle le groupement Z est une substance capable de conférer auxdits polymères une meilleure solubilité ou lipophilie.
xii. Composition pour son application selon xi, dans laquelle les groupements Z sont identiques ou différents et sont choisis dans le groupe comprenant des acides aminés, des acides gras, des alcools gras, des céramides, ou des dérivés de ceux-ci, ou encore des séquences nucléotidiques d'adressage.
xiii. Composition pour son utilisation selon l'un quelconque v à xii dans laquelle les groupements R₉ et R₁₀ sont indépendamment et éventuellement substitués par un groupement Z.
xiv. Composition pour son utilisation selon i ou l'un quelconque des iii à xiv lorsque qu'ils dépendent de i dans laquelle le médicament pour le traitement de lésions tissulaires et/ou de sécheresse tissulaire et/ou de la fibrose.

### Brève description des figures

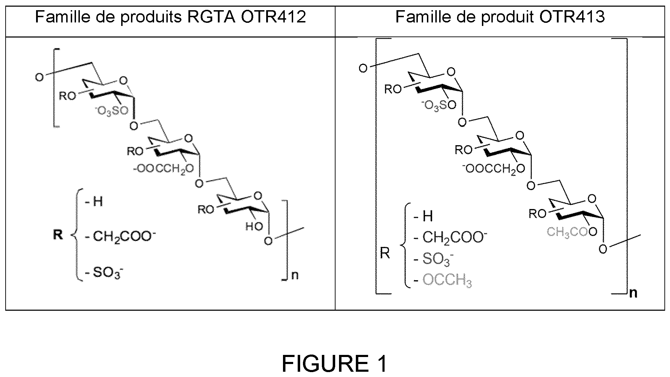

La figure 1 représente des exemples de structure de polymère biocompatible, par exemple la structure des familles de composés OTR412 et OTR413, et OTR415

D'autres avantages pourront encore apparaître à l'Homme du métier à la lecture des exemples ci-dessous, illustrés par les figures annexées, donnés à titre illustratif.

### Exemples

Dans les exemples ci-dessous, différents polymère biocompatibles, également désigné RGTA ont été utilisés. Le tableau 2 ci-dessous résume les différents polymères biocompatibles utilisés dans ces exemples.

**Tableau 2 : résumés des différents polymères biocompatibles (RGTA) utilisés dans ces exemples**

| Polymère biocompatible | | N° des exemples |
|---|---|---|
| Nom du RGTA | P M moyen +/-15% | |
| CMDS OTR41201 | 3 000 | 1,2,6 |
| CMDS OTR41202 | 6 000 | 1,2,6,12 |
| CMDS OTR41203 | 10 000 | 1,6,8,10 |
| CMDS OTR41205 | 20 000 | 1 |
| CMDS OTR41210 | 40 000 | 1 |
| CMDS OTR4120 | 80 000 | 1,2,3,5,7,8,9,11,12,13,14,15,16,17 |
| CMDS OTR4122 | 220 000 | 1,11 |
| CMDS OTR4125 | 500 000 | 1,11 |
| CMDSA OTR41301 | 3 000 | 1,6 |
| CMDSA OTR41302 | 6 000 | 1,6,8,10 |
| CMDSA OTR41303 | 10 000 | 1,6 |
| CMDSA OTR41305 | 20 000 | 1 |
| CMDSA OTR41310 | 40 000 | 1 |
| CMDSA OTR4131 | 80 000 | 1,2,4,5,11,13,14,15,16,17 |
| CMDSA OTR4132 | 220 000 | 1,11 |
| CMDSA OTR4135 | 500 000 | 1,11 |
| CMDSP OTR415 (F6) | 5000 | 18 |

### Exemple 1: Préparation des RGTA - test de stabilité de l'acide hyaluronique en présence de RGTA et de hyaluronidase.

La synthèse des RGTA est largement décrite dans l'art antérieur, par exemple dans le brevet US7396923 intitulé « procédé de sulfonation de composes comprenant des groupements hydroxyles (OH) libres ou des amines primaires ou secondaires » et également dans la référence bibliographique Yasunori I. et al., Biomaterials 2011, 32 :769e776) et Petit E. et al,. Biomacromolecules. 2004 Mar-Apr; 5(2):445-52. [17]:

Dans les exemples ci-dessous, plusieurs RGTA connus et décrits ont été utilisés dont le OTR4120 décrit de nombreuses publications précliniques et cliniques (RGTA®-based matrix therapy - A new branch of regenerative medicine in locomotion. Barritault D, Desgranges P, Meddahi-Pellé A, Denoix JM, Saffar JL. Joint Bone Spine. 2017 May;84(3):283-292. doi: 10.1016/j.jbspin.2016.06.012 [18], RGTA® or ReGeneraTing Agents mimic heparan sulfate in regenerative medicine: from concept to curing patients. Barritault D, Gilbert-Sirieix M, Rice KL, Siñeriz F, Papy-Garcia D, Baudouin C, Desgranges P, Zakine G, Saffar JL, van Neck J. Glycoconj J. 2017 Jun;34(3):325-338. doi: 10.1007/s10719-016-9744-5 [14]. Le composé OTR4131 est un composé comprenant un radical Z qui est un acide gras, à savoir l'acide acétique tel que décrit dans Frescaline G. et al., Tissue Eng Part A. 2013 Jul;19(13-14):1641-53. doi: 10.1089/ten.TEA.2012.0377) [19], Randomized controlled trial demonstrates the benefit of RGTA^{®} based matrix therapy to treat tendinopathies in racing horses.Jacquet-Guibon S, Dupays AG, Coudry V, Crevier-Denoix N, Leroy S, Siñeriz F, Chiappini F, Barritault D, Denoix JM. PLoS One. 2018 Mar 9;13(3):e0191796. doi: 10.1371/journal.pone.0191796 [20] .D'autres composés également décrits dans les documents brevets US06689741, US2014301972A1 dans lequel Z est un acide aminé comme la phénylalanine (Heparan sulfate proteoglycans mediate internalization and propagation of specific proteopathic seeds. Holmes BB, DeVos SL, Kfoury N, Li M, Jacks R, Yanamandra K, Ouidja MO, Brodsky FM, Marasa J, Bagchi DP, Kotzbauer PT, Miller TM, Papy-Garcia D, Diamond MI. Proc Natl Acad Sci U S A. 2013 Aug 13;110(33):E3138-47. doi: 10.1073/pnas.1301440110 [21]) ou autre composé hydrophobe (Structure-activity studies of heparan mimetic polyanions for anti-prion therapies. Ouidja MO, Petit E, Kerros ME, Ikeda Y, Morin C, Carpentier G, Barritault D, Brugère-Picoux J, Deslys JP, Adjou K, Papy-Garcia D. Biochem Biophys Res Commun. 2007 Nov 9;363(1):95-100 [22]).

Différents RGTA sont listés et décrits dans le tableau 1 avec leurs caractéristiques et le tableau 2 précise les numéros exemples dans lesquels ces composés ont été utilisés.

Dans l'exemple ci-dessous différents RGTA ont été testé afin de déterminer leur effet sur l'acide hyaluronique et notamment la détermination d'un éventuel effet protecteur de l'acide hyaluronique vis-à-vis de la hyaluronidase selon le procédé décrit dans de multiples articles, par exemple, In Vitro Evaluation of the Sensitivity of a Hyaluronic Acid PEG Cross-Linked to Bovine Testes Hyaluronidase Nicola Zerbinati et al Open Access Maced J Med Sci. 2018 Jan 25; 6(1):20-24 https://doi.org/10.3889/oamjms.2018 [23], Sall I, Férard G. Comparison of the sensitivity of 11 crosslinked hyaluronic acid gels to bovine testis hyaluronidase. PolymDegrad Stab. 2007; 92: 915-919. https://doi.org/10.1016/j.polymdegradstab.2006.11.020.

Dans cet exemple la Hyaluronidase de type 1-S (EC 3.2.1.35) provenait de testicule de bovins (sigma Aldrich H3506) et a été diluée dans du tampon phosphate salin (PBS) à pH 7,2 pour obtenir une solution à 5000 U/mL.

0,3mL d'un gel de 20mg/mL de d'acide hyaluronique de strotococcus zooepidermicus (sigma Aldrich H39390) a été déposé au fond de tubes 5 de mL et on ajoute 25 µL de RGTA à différentes concentrations puis incubé à 37°C la nuit.

125 µL de la solution d'enzyme sur la surface du gel ont été ajouté et les tubes ont été incubés pendant 2 jours (h), La réaction a été arrêtée par addition de 0,1 mL de solution de tetraborate de potassium 0,8 mol/L Ph 9,1 suivi d'une agitation au vortex suivi d'un chauffage de 3 minutes à 100°C.

La mesure de l'activité de l'enzyme par le relargage de N-acétyl-D-glucosamine (NAG), a été effectuée par la méthode de Reissig et al (Reissig JL, Strominger JL, Leloir LF. A modified colorimetric method for the estimation of N-acetylamino sugars. J Biol Chem 1955;217:959e96 [25].

Brièvement une solution du réactif de Ehrlich (Sigma Aldrich) diluée au 1/10 dans de l'acide acétique est ajoutée à chaque tube (3 mL) qui sont ensuite après vortex agitation au vortex incubée à 37° pendant 20 minutes, puis les tubes sont centrifugés à 1000 g pendant 15 minutes et le surnagent est mesuré à 585 nm contre du PBS+ réactif de Ehrlich.

Le tableau 3 ci-dessous résume les résultats obtenus pour une quantité d'acide hyaluronique (300µL à 20mg/mL ) constante non modifié (PM de 250 000 Daltons en présence de 125 µL de hyaluronidase et de dose croissante de composé de deux familles de RGTA de différentes tailles moléculaires allant de 3000 D à 500 000 D décrits dans le tableau 1.

**Tableau 3 : résultats de la dégradation de l'acide hyaluronique en présence de polymère biocompatibles et de l'hyaluronidase**

| HA non modifié | RGTA: 25µL | RGTA | | | | | | | |
|---|---|---|---|---|---|---|---|---|---|
| | | OTR | | | | | | | |
| | (µg/mL) RGTA | 4120 | 41201 | 41202 | 41203 | 41205 | 41210 | 4122 | 4125 |
| | | % dégradation de l'AH par l'hyaluronidase +/_5% | | | | | | | |
| 300µL de gel de HA à 20 mg/mL) | 50 | 5% | 4% | 5% | 5% | 5% | 5% | 5% | 5% |
| | 10 | 5% | 9% | 6% | 6% | 5% | 5% | 5% | 5% |
| | 1 | 40% | 45% | 46% | 48% | 44% | 40% | 40% | 40% |
| | 0 | 100% | 100% | 100% | 100% | 100% | 100% | 100% | 100% |
| RGTA | | 4131 | 41301 | 41302 | 41303 | 41305 | 41310 | 43132 | 4135 |
| 300µL de gel de HA à 20 mg/mL) | 50 | 3% | 5% | 5% | 5% | 5% | 5% | 5% | 5% |
| | 10 | 4% | 8% | 7% | 6% | 5% | 5% | 5% | 5% |
| | 1 | 38% | 45% | 44% | 44% | 42% | 40% | 40% | 40% |
| | 0 | 100% | 100% | 100% | 100% | 100% | 100% | 100% | 100% |

Légende du tableau 3 : 300 µL de gel +25µL RGTA+ 100 µL (800U) Enzyme incubés 2j à 37°C. Arrêt par 0,1 mL de potassium tetraborate (0.8mol/L, PH 9.1) Mélange vigoureux vortex, Chauffage 3min à 100°C et ajout de 3mL de réactif Ehrlich et lecture à 585nM.

Tel que démontré dans le tableau 3 ci-dessus les RGTA a un effet inhibiteur de l'activité de la hyaluronidase et/ou protecteur de l'acide hyaluronique. Cet exemple démontre également clairement que l'effet est dose dépendant. La dose à 25µL de RGTA à 10 µg/mL est aussi efficace que celle de 50µg/mL. Cet exemple démontre également clairement et avantageusement qu'une faible concentration (1 µg/mL de polymère biocompatible (RGTA) permet avantageusement une inhibition de plus de 60% de la dégradation de l'acide hyaluronique par la hyaluronidase. Cet exemple montre également qu'il n'y a pas de différence significative dans l'effet protecteur des RGTA contre une dégradation par la hyaluronidase selon le poids moléculaire des RGTA ni entre le RGTA carboxylméthyl dextran sulfate à savoir OTR41201, OTR41202, OTR41203, CMDS OTR41205, CMDS OTR41210, CMDS OTR4120, CMDS OTR4122, CMDS OTR4125, ou le RGTA carboxylmethyl dextran sulfate acetylés, à savoir OTR41301, CMDSA OTR41302, CMDSA OTR41303, CMDSA OTR41305, CMDSA OTR41310, CMDSA OTR4131, CMDSA OTR4132, CMDSA OTR4135 décrits dans le tableau 1 ci-dessus

### Exemple 2 : Effet synergique des RGTA et des HA sur la synthèse des collagènes, sur la réduction de l'index fibrotique et sur la polarité de la sécrétion des collagènes

Les RGTA sont également connu pour moduler l'expression des collagènes et la fibrose (US06689741, US2014301972A1 [4]) dans la peau de nombreux articles décrivent ces propriétés notamment dans le traitement de la fibrose cutanée (Garcia-Filipe et al. 2007 [8]), les tissus musculaires (A substituted dextran enhances muscle fiber survival and regeneration in ischemic and denervated rat EDL muscle. Desgranges P, Barbaud C, Caruelle JP, Barritault D, Gautron J. FASEB J. 1999 Apr;13(6):761-6 [26]), cardiaques (New agents for the treatment of infarcted myocardium. Yamauchi H, Desgranges P, Lecerf L, Papy-Garcia D, Tournaire MC, Moczar M, Loisance D, Barritault D. FASEB J. 2000 Nov;14(14):2133-4), osseux (Barritault et al 2017 [18]) ou digestifs (Alexakis et al, 2004 [5]). L'effet d'une éventuelle combinaison des RGTA avec de l'AH n'a cependant pas été étudié et n'est pas connu

Des fibroblastes dermiques humains ont été mis en culture dans des puits de plaques 24 trous selon les protocoles décrits selon Garcia-Filipe S et al 2007 [8]

Les fibroblastes ensemencés à 10 000 cellules par puits sont cultivés pendant 5 jours pour atteindre la confluence avec 90 000cellules par puits, puis mis dans un milieu sans sérum pendant 24h en présence de 15mCi /mL de 5-3H Proline et 50 mg/mL d'acide ascorbique en présence ou absence de RGTA OTR4120 ou OTR4131 à 10 et 100 µg/mL et d'AH (20mg/mL) selon le protocole ci-dessous.

Le milieu de culture et les cellules sont séparément récoltés après 24h, dialysés contre de l'eau, digérés par de la pepsine et les phénotypes de collagènes sont analysés après séparation par électrophorèse en gel SDS selon le procédé décrit dans Asselot-Chapel C, et al Expression of fibronectin and interstitial collagen genes in smooth muscle cells: modulation by low molecular weight heparin fragments and serum.Biochem Pharmacol. 1995 Mar 1;49(5):653-9 [28].

Les zones correspondantes aux collagènes 1 et 3 ont été découpées du gel et le tritium est compté. Le % du collagène total cellulaire est le rapport entre la radio activité dans la couche cellulaire sur la radioactivité dans le surnagent.

**Le tableau 4 ci-dessous résume les résultats obtenus**

| RGTA | RGTA 100µg/mL | A.H. 20mg/mL | COL3 : dpm ³H/1000cellules (+/-10%) | COL1 dpm ³H/1000 cellules (+/-10%) | COL3/ COL1* | % total COL cellulaire |
|---|---|---|---|---|---|---|
| | - | - | 170 | 720 | 0,23 | 7 |
| | - | + | 170 | 960 | 0,177 | 7 |
| Héparine | | + | 100 | 600 | 0,166 | 15 |
| CMDS OTR4120 | + | - | 60 | 720 | 0,083 | 30 |
| CMDS OTR4120 | + | + | 60 | 1100 | 0,054 | 45 |
| CMDS OTR4120 1 | + | - | 45 | 720 | 0,062 | 8 |
| CMDS OTR4120 1 | + | + | 45 | 1100 | 0,041 | 35 |
| CMDS OTR4120 2 | + | - | 45 | 720 | 0,062 | 6 |
| CMDS OTR4120 2 | + | + | 45 | 1100 | 0,041 | 35 |
| CMDSA OTR4131 | + | - | 60 | 730 | 0,082 | 30 |
| CMDSA OTR4131 | + | + | 60 | 1180 | 0,050 | 45 |

| | | | | | | |
|---|---|---|---|---|---|---|
| * Une mesure dans une peau normale de souris par des techniques comparables donne un rapport COL1 sur COL3 de 10 (figure 1 de Garcia-Filipe S et al, 2007 [8]). | | | | | | |

Tel que démontré dans le tableau 4 ci-dessus, les RGTA ont un effet sur la régulation de la synthèse des collagènes en diminuant la quantité de collagène 3 par près de 3 fois (de 170 à 60, ce qui se traduit un vivo par une réduction de la fibrose) ainsi que l'effet de l'AH sur une augmentation de 20% (720 contre 960) de la synthèse du collagène 1.

Tel que démontré dans le tableau 4 ci-dessous, la combinaison de polymère et d'acide hyaluronique a, de manière inattendue, un effet synergique pour la synthèse de COL1 avec une augmentation de 40% (720 à 1100).

Tel que démontré dans le tableau 4 ci-dessous, la combinaison de polymère et d'acide hyaluronique a en outre, de manière inattendue, un effet synergique sur la polarisation de la sécrétion du collagène au niveau des cellules et leur matrice. Le % de collagène passe de 7% à 45% soit 6,4 fois supérieur, par rapport à l'ensemble du Collagène total cumulé dans l'espace cellulaire et péri-cellulaire.

Tel que démontré dans cet exemple, la composition comprenant un polymère de formule AaXxYy ou AaXxYyZz et de l'acide hyaluronique permet avantageusement et de manière synergique une augmentation de la synthèse de collagène, une amélioration de la synthèse de collagène et permet avantageusement de recréer l'espace matriciel favorable à une bonne différenciation cellulaire et tissulaire

### Exemple 3 : Effet synergique des RGTA et des HA en utilisation ex-vivo sur des membranes tympaniques, cornéens, biomatériaux et dans des procédés d'impression 3D de tissues ou d'organes

### 3-1 Préincubation de membranes tympaniques et des cornéens avant transplantation/conservation d'organe

Dans cet exemple l'OTR4120 a été utilisé à 10µ/mL dans une solution de 5mg/mL d'AH (de qualité injectable) commercial, provenant notamment de différents fournisseurs pour imprégner pendant quelques minutes le greffon, par exemple la cornée, ou le tympan. L'imprégnation est effectuée avant d'effectuer la greffe, puis à la fin de l'opération quelques gouttes de la même solution sont ajoutées.

De l'avis des chirurgiens la prise et la récupération fonctionnelle étaient de bien meilleure qualité que celle observée selon les protocoles antérieurs.

### 3-2 Pré-incubation de biomatériaux pour comblement osseux ou cartilagineuse avec le produit de l'invention.

Dans cet exemple plusieurs biomatériaux substituts osseux utilisés pour la reconstruction osseuse, cartilagineuses ou des disques intervertébraux ont été imprégnés par une solution comprenant la combinaison selon l'invention, à savoir une combinaison de polymère de formule AaXxYy ou AaXxYyZz et de l'AH avant d'être implantés.

Dans cet exemple, la solution d'imprégnation utile comprend de l'OTR4120 à une concentration de 10 µg/mL et une concentration d'acide hyaluronique de 1mg/mL

Les biomatériaux imprégnés étant des poudres d'os décellularisés, ou des produits de synthèse comme des céramiques, notamment des phosphate tricalciques ou TCP, ou de l' hydroxyapatite HA, ou des biomatériaux composites comprenant en outre des protéines comme le collagène.

### 3-3. Utilisation d'une composition comprenant une combinaison de polymère de formule AaXxYy ou AaXxYyZz et de l'acide hyaluronique (AH) comme encre dans des procédés d'impression 3D de tissues ou d'organes

Dans cet exemple, la composition utile comprend de 10 à une concentration de 100µg/mL et une concentration d'acide hyaluronique de 1 à 20mg/mL selon des méthodes ou procédés d'impression décrites dans différentes revues notamment Jammed Microgel Inks for 3D Printing Applications. Highley CB, Song KH, Daly AC, Burdick JA. Adv Sci (Weinh). 2018 Oct 24;6(1):1801076. doi: 10.1002/advs.201801076. [33], Collagen/heparin sulfate scaffolds fabricated by a 3D bioprinter improved mechanical properties and neurological function after spinal cord injury in rats. Chen C, Zhao ML, Zhang RK, Lu G, Zhao CY, Fu F, Sun HT, Zhang S, Tu Y, Li XH. J Biomed Mater Res A. 2017 May; 1 05(5): 1324-1332. doi: 10.1002/jbm.a.36011. [34] Development and Evaluation of Hyaluronic Acid-Based Hybrid Bio-Ink for Tissue Regeneration. Lee J, Lee SH, Kim BS, Cho YS, Park Y. Tissue Eng Regen Med. 2018 Sep 21;15(6):761-769. doi: 10.1007/s13770-018-0144-8. [35]

Les résultats obtenus ne sont pas présentés ici.

Tel que démontré dans l'exemple ci-dessus, il apparait clairement que la composition comprenant un polymère de formule AaXxYy ou AaXxYyZz et de l'acide hyaluronique permet l'obtention d'effet synergique, notamment in vitro, permettant avantageusement de recréer l'espace matriciel favorable à une bonne différenciation cellulaire et tissulaire. En outre cet exemple démontre clairement que cet effet peut être obtenu pour toutes les applications et/ou tissu nécessitant la production et/ou une reformation et/ou une augmentation de la matrice extracellulaire.

Cet exemple démontre également clairement, notamment de par la stimulation de la production de composants de la matrice extracellulaire que la composition comprenant un polymère de formule AaXxYy ou AaXxYyZz et de l'acide hyaluronique permet en outre avantageusement de protéger l'acide hyaluronique de sa dégradation et de plus de prolonger les effets du HA dans ses utilisations de comblement et d'hydratation tissulaire.

Il apparait également clairement que la composition comprenant un polymère de formule AaXxYy ou AaXxYyZz et de l'acide hyaluronique permet une amélioration de l'organisation et de la répartition et la polarisation de la production des constituants matriciels comme les collagènes permettant avantageusement une amélioration de la qualité de la matrice extracellulaire et du tissu traité en général, quelques soient les tissus ou organes considérés.

Il apparait également clairement que la composition comprenant un polymère de formule AaXxYy ou AaXxYyZz et de l'acide hyaluronique permet lors d'une lésion ou microlésion d'un tissu biologique une substitution à la fois des héparanes sulfates et de l'acide hyaluronique détruits lors de la lésion par respectivement le polymère de formule AaXxYy ou AaXxYyZz et de l'acide hyaluronique présents dans la composition selon l'invention.

En outre, lors de l'injection de la composition selon l'invention une éventuelle destruction de l'espace matriciel peut avoir lieu, sur le site même de la pénétration de la composition puis s'étendre par la diffusion de la composition injectée, occasionnant des microlésions associées au remplissage de l'espace de comblement. Ces micro-lésions peuvent induire une réaction locale d'inflammation et une destruction du micro environnement et de l'architecture matricielle. Le polymère de formule AaXxYy ou AaXxYyZz peut alors se substituer aux héparanes sulfates endogènes dont la destruction peut être activée en réponse à cette agression tissulaire par la réponse tissulaire inflammatoire et ainsi aider à la reconstruction de l'architecture matricielle tandis que l'acide hyaluronique remplit l'espace du micro environnement. Les glycanases induites par la lésion peuvent être activées mais sont avantageusement inhibées par le polymère de formule AaXxYy ou AaXxYyZz permettant en outre avantageusement une réduction et permettre une durée prolongée du comblement tissulaire. De plus tel que démontré la composition comprenant un polymère de formule AaXxYy ou AaXxYyZz et de l'acide hyaluronique ayant un effet synergique et inattendu sur les cellules fibroblastiques dermiques permet avantageusement d'augmenter la synthèse et la quantité de Collagène 1 (COL1), et un renforcement de la matrice tissulaire quelques soient les tissus.

Cet exemple démontre donc clairement que la composition comprenant un polymère de formule AaXxYy ou AaXxYyZz et de l'acide hyaluronique permet avantageusement un renforcement de la fonction barrière de la peau, permet une amélioration de la qualité et de l'aspect de la peau, notamment en améliorant son hydratation, épaisseur, souplesse .... En outre, cet exemple démontre également clairement que l'effet est durable, notamment de par l'inhibition des enzymes responsables de la dégradation du collagène mais également par l'induction/la stimulation de la synthèse de collagène.

De plus cet exemple démontre également clairement que la composition comprenant un polymère de formule AaXxYy ou AaXxYyZz et de l'acide hyaluronique permet avantageusement de recréer une matrice extracellulaire de meilleure qualité qui a son tour va induire indirectement une réponse cellulaire de meilleure qualité. Cet effet en boucle va prolonger la durée bénéfique du traitement et de l'effet anti-âge

### Exemple 4 : utilisation d'une composition comprenant un polymère de formule AaXxYy ou AaXxYyZz et de l'acide hyaluronique pour le traitement du vieillissement cutanée

Dans cet exemple, des compositions cosmétiques comprenant du RGTA OTR4131 et de l'acide hyaluronique ont été utilisés par application topique. En particulier, une crème de jour référencée FOR075.039 contenant 1µg/mL de RGTA OTR4131 avec 20mg/mL d'acide Hyaluronique (PM-400 à 700kD), une crème de nuit référencée FOR075.040 contenant 1µg/mL de RGTA OTR4131 avec 20mg/mL d'acide Hyaluronique (PM-400 à 700kD) et un sérum référencé FOR075.041 contenant 2 µg/mL de RGTA OTR4131 avec 40mg/mL d'acide Hyaluronique PM-400 à 700kD) ont été utilisés. L'acide Hyaluronique provenant de Biphil. (http://www.biophilgroup.com/).

Les tableaux 5 à 7 résumes les caractéristiques des compositions utilisées, le polymère biocompatible de formule AaXxYyZz utilisé est désigné par Sodium carboxymethyl dextran acétate sulfate dans ces tableaux :

**Tableau 5 : formulation crème de Jour**

| **Dénomination INCI EU** | **% (m/m) dans le produit** |
|---|---|
| Aqua | 66,6899 |
| Caprylic/Capric triglyceride | 8,5000 |
| Glycerin | 8,5000 |
| Pentylene glycol | 5,0000 |
| Coco-caprylate | 3,4984 |
| Coconut alkanes | 2,8000 |
| Hydroxyethyl acrylate/sodium acryloyldimethyl taurate copolymer | 1,7000 |
| Acrylates copolymer | 1,4100 |
| Coco caprylate/caprate | 0,7000 |
| Parfum | 0,5000 |
| Ethylhexylglycerin | 0,3000 |
| Sodium hyaluronate | 0,2000 |
| Sorbitan isostearate | 0,1100 |
| VP/Polycarbamyl polyglycol ester | 0,0500 |
| Hydrolyzed sesame protein pg-propyl methylsilanediol | 0,0400 |
| Tocopherol | 0,0016 |
| Sodium carboxymethyl dextran acetate sulfate | 0,0001 |

**Tableau 6 : formulation crème de nuit**

| **Dénomination INCI EU** | **% (m/m) dans le produit** |
|---|---|
| Aqua | 53,4999 |
| Helianthus annuus seed oil | 10,0000 |
| Octyldodecyl myristate | 8,0000 |
| Coconut alkanes | 6,4000 |
| Glycerin | 5,0000 |
| Pentylene glycol | 5,0000 |
| Glyceryl stearate citrate | 4,0000 |
| Zea mays starch | 2,5000 |
| Glyceryl stearate | 2,0000 |
| Coco caprylate/caprate | 1,6000 |
| Tocopheryl acetate | 1,0000 |
| Ethylhexylglycerin | 0,3000 |
| Xanthan gum | 0,3000 |
| Parfum | 0,2000 |
| Sodium hyaluronate | 0,2000 |
| Sodium carboxymethyl dextran acetate sulfate | 0,0001 |

**Tableau 7 : formulation sérum**

| **Dénomination INCI EU** | **% (m/m) dans le produit** |
|---|---|
| Aqua | 82,1598 |
| Glycerin | 6,4000 |
| Pentylene glycol | 5,0000 |
| Caprylic/Capric triglyceride | 2,0000 |
| Helianthus annuus seed oil | 2,0000 |
| Sodium acrylates copolymer | 0,7000 |
| Polyglyceryl-3 methylglucose distearate | 0,5000 |
| Sodium hyaluronate | 0,4000 |
| Lecithin | 0,3000 |
| Ethylhexylglycerin | 0,3000 |
| Parfum | 0,2000 |
| Terminalia ferdinandiana fruit extract | 0,0400 |
| Sodium carboxymethyl dextran acetate sulfate | 0,0002 |

Cette étude a été réalisée sur une population cible de femmes d'âge moyen de 62±1ans (de 47 et 67 ans) et dont le type de peau et photo type se répartissent ainsi 50% avaient une peau mixte, 9% Normale et 43% sèche répartie en deux phototypes : type 2 pour 59% et type 3 pour 41%.

L'application et la fréquence d'application sont décrites dans le tableau 8 ci-dessous. L'analyse des résultats a été effectué avec le Système 3D Primo (marque de commerce) Lite, le Conomètre (marque de commerce), DUB (marque de commerce) Skin Scanner Conomètre (marque de commerce) : et des scores cliniques des questionnaires, recueil des éventuelles

**Tableau 8 : protocole d'application et d'utilisation des compositions**

| | **Zones** | **Frequence** | **Mode** |
|---|---|---|---|
| **Le Jour - FOR075.039** | A domicile : visage et décolleté | A domicile : une fois par jour (le matin) avant le maquillage, après la toilette et l'application du Sérum. Au laboratoire : une application unique standardisée (2 µl/cm²) en synergie avec deux autres produits. | A domicile : appliquer au niveau du visage par un léger massage jusqu'à pénétration complète. |
| **La Nuit - FOR075.040** | | A domicile : une fois par jour (le soir) après la toilette. | Au laboratoire : massage léger et uniforme avec un doigtier. |
| | | Au laboratoire : une application unique standardisée (2 µl/cm²) en synergie avec deux autres produits. | |
| | Au laboratoire : zone traitée définie au niveau des jambes (concerne 11 volontaires uniquement). | | |
| **Le Sérum - FOR075.041** | | A domicile : une fois par jour (le matin) après la toilette et avant la crème jour. | A domicile : appliquer au niveau du visage par un léger massage jusqu'à pénétration complète, en insistant sur la zone des rides et zone avec des taches. |
| | | Au laboratoire : une application unique standardisée (2 µl/cm²) en synergie avec deux autres produits. | |
| | | | Au laboratoire : massage léger et uniforme avec un doigtier. |

Dans cet exemple, les effets suivants des compositions ont été étudiés :
- Effet anti-âge ;
- Effet lissant ;
- Effet détoxifiant ;
- Effet sur la fermeté, l'élasticité et la souplesse cutanées ;
- Effet redensifiant / repulpant ;
- Effet hydratant.

En particulier, une évaluation des paramètres suivant a été réalisée :
- l'effet lissant / anti-rides des produits étudiés par des mesures à l'aide du système 3D Primos^{®}
- Lite ;
- leur effet détoxifiant par scorage clinique effectué par le technicien en charge de l'étude ;
- leur effet sur les propriétés biomécaniques de la peau par des mesures à l'aide du Cutomètre^{®} ;
- leur effet redensifiant par des mesures de densité du derme à l'aide du DUB^{®} SkinScanner ;
- leur effet hydratant deux et quatre heures après une application unique standardisée au
- laboratoire par des mesures à l'aide de Cornéomètre^{®} (concerne uniquement 11 volontaires) ;
- réaliser et collecter des empreintes de peau en silicone polymérique Silflo^{®}replicas
- subjectivement l'appréciation de leur acceptabilité cosmétique, de leur efficacité et de leur
- utilisation ultérieure par analyse des réponses données par les volontaires à un questionnaire
- d'évaluation subjective.
- recueillir les éventuelles réactions indésirables.

Les résultats obtenus sont résumés dans les tableaux 9 à 13 suivants :

**Tableau 9 : résultats après application de la composition**

| Paramètres | Cinétique | Δ (moyenne ± écart-type | Variations entre les cinétiques (moyenne ± SEM) | | | Type d'analyse statistiqu | p | Significativité | % de volontaires présentant |
|---|---|---|---|---|---|---|---|---|---|
| Eclat et luminosité de la peau | ΔJ7 | 23% | 0,9 | ± | 0,1 | Wilcoxon | <0,0001 | Oui | 86% |
| | ΔJ28 | 42% | 1,6 | ± | 0,1 | Wilcoxon | <0,0001 | Oui | 100% |
| Uniformité du teint | ΔJ7 | 5% | 0,2 | ± | 0,1 | NA | NA | NA | 27% |
| | ΔJ28 | 34% | 1,2 | ± | 0,2 | Wilcoxon | <0,0001 | Oui | 86% |
| Grain de peau | ΔJ7 | 10% | 0,4 | ± | 0,1 | NA | NA | NA | 32% |
| | ΔJ28 | 38% | 1,3 | ± | 0,2 | Wilcoxon | <0,0001 | Oui | 86% |
| Fraîcheur de la peau | ΔJ7 | 27% | 1,0 | ± | 0,1 | Wilcoxon | <0,0001 | Oui | 91% |
| | ΔJ28 | 47% | 1,7 | ± | 0,2 | Wilcoxon | <0,0001 | Oui | 95% |

**Tableau 10 : Variation des paramètres du relief cutané en comparaison à l'état initial**

| | | | | Analyse statistique | | | |
|---|---|---|---|---|---|---|---|
| | Cinétiques | Δ (moyenne ± écart-type) | Δ% sur la moyenne | p | significatif | Test | % de volontaires présentant l'effet attendu |
| Rugosité moyenne : Ra (en µm) | Δ J28 | -0,5 ± 0,2 | -3% | 0,034 | Oui | Test t | 68% |
| Relief moyen : Rz (en µm) | Δ J28 | -2,2 ± 1,0 | -3% | 0,041 | Oui | Test t | 68% |

**Tableau 11 : Variation des propriétés biomécaniques de la peau après 28 jours d'utilisation quotidienne (en comparaison à l'état initial)**

| | | | COMPARAISON AVANT / APRES SUR LA ZONE TRAITEE PAR TROIS | | | | |
|---|---|---|---|---|---|---|---|
| | | Cinétiques | ΔJX-J0 en mm (moyenne ± écart-type) | Test t de Student | | | |
| | | | | p | Significativité | % d'efficacité | % de volontaires présentant l'effet attendu |
| FERMETE | Paramètre R0 (soit Uf) | Δ J28 | -0,021 ± 0,012 | 0,088 | Oui | 6% | 45% |
| ELASTICITE BRUTE | Paramètre R7 (soit Ur/Uf) | Δ J28 | 0,116 ± 0,015 | <0,001 | Oui | 47% | 91% |
| TONICITE | Paramètre Ur (soit R7*R0) | Δ J28 | 0,035 ± 0,006 | <0,001 | Oui | 39% | 73% |

**Tableau 12 : Variation du taux d'hydratation cutanée après une application unique standardisée des produits en synergie (en comparaison à une zone non traitée)**

| **Paramètre** | **Cinétiques** | ΔΔ **Tx-T0** (moyenne ± SEM) | **Statistiques** | | **% d'efficacité** | **% de volontaires présentant une amélioration** |
|---|---|---|---|---|---|---|
| | | | p= | Significativité | | |
| **Taux d'hydratation cutanée** | Δ **t2h** | 17 ± 1 | **<0,001** | **Oui** | **80%** | **100%** |
| | Δ **t4h** | 20 ± 1 | **<0,001** | **Oui** | **90%** | **100%** |

**Tableau 13 : résumé des résultats obtenus**

| Paramètres | Résultats |
|---|---|
| Hydratation cutanée | - T2H: Amélioration moyenne de 80% chez 100% des utilisatrices. |
| | - T4H: Amélioration moyenne de 90% chez 100% des utilisatrices. |
| Antirides | J28: Baisse moyenne de 3% du relief et de la rugosité de la peau sur 68% des utilisatrices |
| Eclat et luminosité | - J7: Amélioration moyenne de 23% chez 86% des utilisatrices. |
| | - J28: Amélioration moyenne de 42% chez 100% des utilisatrices. |
| Uniformité du teint | J28: Amélioration moyenne de 34% chez 86% des utilisatrices. |
| Grain de la peau | J28: Amélioration moyenne de 38% chez 86% des utilisatrices. |
| Fraicheur de la peau | - J7: Amélioration moyenne de 27% chez 91% des utilisatrices. |
| | - J28: Amélioration moyenne de 47% chez 95% des patientes. |
| Elasticité | J28: Amélioration moyenne de 47% chez 91% des patientes. |
| Tonicité | J28: Amélioration moyenne de 39% chez 73% des utilisatrices |

Tel que démontré dans les tableaux ci-dessus, des exemples de compositions selon l'invention ont permis avantageusement d'augmenter l'hydratation cutanée, à un effet antiride, améliore l'éclat et la luminosité de la peau, l'uniformité du teint, le grain de la peau, la fraicheur de la peau, l'élasticité et la tonicité de la peau.

**Le tableau 14 ci-dessous comprend les résultats obtenus et démontre l'efficacité de la composition utilisée.**

| Propriétés | Favorable |
|---|---|
| Appréciation générale | |
| Agréable d'utilisation | 100% |
| Aspect plaisant | 100% |
| Pénètre rapidement | 100% |
| Appréciation du Parfum | 91% |
| Laisse la peau non-grasse | 100% |
| Laisse la peau non-grasse et non-collante | 96% |

| Efficacité perçue par l'utilisatrice | |
|---|---|
| Laisse la peau douce | 100% |
| Rend la peau souple | 100% |
| Rend la peau hydratée et nourrie | 100% |
| Régénère la peau | 96% |
| Lisse les rides et ridules | 95% |
| A un effet raffermissant | 96% |
| Ravive l'éclat du teint | 95% |
| Améliore la texture de la peau | 96% |
| mon entourage me trouve plus jeune | 86% |

Aucune réaction indésirable n'a été observée pendant l'étude et les compositions ont été appréciées par la majorité des volontaires pour ses caractéristiques et son efficacité. En effet plus de 95% des sujets souhaitent continuer à utiliser les compositions testées.

Tel que démontré dans ci-dessus, des exemples de compositions selon l'invention ont permis avantageusement d'augmenter synergiquement l'hydratation cutanée, à un effet antiride, améliore l'éclat et la luminosité de la peau, l'uniformité du teint, le grain de la peau, la fraicheur de la peau, l'élasticité et la tonicité de la peau.

En outre, cet exemple démontre clairement que des exemples de composition selon l'invention comprenant un polymère de formule AaXxYy ou AaXxYyZz et de l'acide hyaluronique permet avantageusement d'améliorer l'aspect de la peau, a un effet antivieillissement cutané, notamment un effet antiride et permet de lisser la peau. En outre, cet exemple démontre clairement que des exemples de composition selon l'invention comprenant un polymère de formule AaXxYy ou AaXxYyZz et de l'acide hyaluronique permet avantageusement de détoxifier la peau tout en augmentant et améliorant ces propriétés mécaniques, par exemple son élasticité, sa souplesse.

En outre, cet exemple démontre clairement que des exemples de composition selon l'invention comprenant un polymère de formule AaXxYy ou AaXxYyZz et de l'acide hyaluronique permet avantageusement d'améliorer l'hydratation de la peau.

### Exemple 5 : Effet sur le comblement des rides par injection intradermique ou sous cutanées du mélange RGTA et AH

Une utilisation du RGTA 4120 ou OTR 4131 par injection sous cutanée et de l'injection de l'AH pour la réduction des rides et sur la durée de cet effet ont été effectués par des médecins et/ou des patients.

Les administrations ont été réalisées à la seule initiative de médecins ou de patients pour eux même et par eux même (le patient pouvant être médecin) ou par moi-même sur moi-même en utilisant des produits à base de OTR4120 ou OTR4131 stérile disponibles sur le marché et de AH également disponible (selon les pays par internet ou autre) parfois à partir échantillons originalement destinés à la recherche.

Dans tous les cas ces usages étaient hors indication, les deux produits RGTA et des HA de différentes sources ont été administrés dans des combinaisons et selon des modes opératoires différents indiqués et les effets sont résumés ci-dessous.

Plusieurs formulations et modes opératoires différents ont été mis en œuvre parfois par les patients eux même dans différentes zones de rides (notamment pattes d'oie, rides du front, rides du Lion, rides péribuccales, sillons naso-géniens, sillons labio- mentonniers, poches sous les yeux, rides du cou etc..

Les modes d'administrations étaient indépendamment les suivant :
- Co-injection d'un mélange d'acide hyaluronique AH et RGTA OTR4120 ou 4131 a été réalisé dans la seringue avant injection (pouvant être réitéré à 1mois), ou
- Injection de AH suivie d'injection après 24h ou 48h du mélange de RGTA (OTR4120 ou 4131) et AH

Le RGTA OTR 4120 ou OTR 4131 étant en solution stérile dans du sérum physiologique respectivement à 100 µg/mL (en flacons de 5 mL) et à 10 µg/mL(en flacon de 1 mL)

Les volumes injectés de RGTA seul ne dépassent pas 1mL par site d'injection

Le mode d'injection étant réalisé en utilisant des aiguilles de type insulines ou en mésothérapies

Acide Hyaluronique ou AH est de différentes origines naturel de différentes tailles ou réticulé et accessibles sur le marché librement selon les pays (via des sites internet, ou en pharmacie ou via des réseaux de distribution auprès des médecins

Le AH est en solution entre 0.5 et 25 mg/mL, de préférence entre 10 et 20 mg/mL

L'effet sur les rides est évalué par la satisfaction de l'individu traité.

Dans tous les cas une amélioration est constatée dès les 15 jours avec un effet visible et la disparition de signes d'inflammation (rougeur, douleur) associés aux processus d'injection. Cette rapidité d'effet est également un effet surprenant, en particulier la disparition rapide des signes d'inflammation.

A 1 mois mais également à 6 mois les effets se confirment et la satisfaction est bonne dans tous les traitements. Cet effet sur la durée est inhabituel la durée et observé même avec des AH naturels de hauts poids moléculaire et non réticulé.

Cet exemple démontre donc clairement qu'un exemple de composition selon l'invention comprenant un polymère de formule AaXxYy ou AaXxYyZz et de l'acide hyaluronique permet avantageusement d'améliorer l'aspect de la peau et peut être utile pour le comblement de rides et/ou ridules. En outre, cet exemple démontre clairement qu'un exemple de composition selon l'invention comprenant un polymère de formule AaXxYy ou AaXxYyZz et de l'acide hyaluronique permet avantageusement d'obtenir un effet rapide, sans effet secondaires indésirables, tout en limitant une éventuelle réaction inflammatoire.

Cet exemple démontre clairement qu'un exemple de composition selon l'invention comprenant un polymère de formule AaXxYy ou AaXxYyZz et de l'acide hyaluronique permet avantageusement d'améliorer l'aspect de la peau et peut être utile pour le comblement de rides et/ou ridules sans nécessiter d'application multiples et/ou répétée de la composition.

Enfin cet exemple démontre clairement qu'un exemple de composition selon l'invention comprenant un polymère de formule AaXxYy ou AaXxYyZz et de l'acide hyaluronique permet avantageusement d'améliorer l'aspect de la peau et peut être utile pour le comblement de rides et/ou ridules de manière durable, par exemple pendant 6 mois après application.

### Exemple 6 - Utilisation de polymères de formule AaXxYy ou AaXxYyZz et de l'acide hyaluronique contre le vieillissement cutané

Dans cet exemple, des compositions pour application topique sont préparées. Il s'agit de formulation topique comprenant du OTR41201 ou OTR41202 ou OTR41203 ou encore de la famille des OTR413101, OTR413102, OTR413103 à des doses de 0,1 1, 10 ou 100 µg/mL de produit final, en particulier des compositions pour application topique sont préparées comprenant du OTR41201 ou OTR41202 à des doses comprises de 1 à 10 µg/mL avec ou sans acide hyaluronique à des concentrations allant de 0,1 mg/mL de préférence 20 mg/mL de préférence 10 mg/mL

Les formulations de crème correspondent à celle utilisées dans l'exemple 4.

Ces compositions sont utiles comme application seule mais aussi en complément d'application et/ou du traitement des comblements de rides par une composition comprenant un polymère de formule AaXxYy ou AaXxYyZz et de l'acide hyaluronique tel que décrit dans l'exemple 5 ci-dessus.

Dans cet exemple, après comblement de rides tels que décrit dans l'exemple 5 ci-dessus, la composition topique précitée comprenant du OTR41201 ou OTR412002 à des doses de 0,1, 1, 10 ou 100 µg/mL de produit final, en particulier des compositions pour application topique sont préparées comprenant du OTR41201 ou OTR412002 à des doses comprises de 1 à 10 µg/mL avec de l'acide hyaluronique à des concentrations de 0,1 mg/mL de préférence 20 mg/mL est appliqué sur la zone qui a été traitée préalablement.

Avantageusement, le polymère de formule AaXxYy ou AaXxYyZz utilisé, à savoir OTR41201 ou OTR41202 présente un poids moléculaire faible qui permet avantageusement de franchir la barrière de l'épiderme et peut pénétrer dans la zone du derme et va renforcer l'effet local de la composition préalablement administrée. Par exemple elle permet avantageusement d'augmenter encore la sécrétion des collagènes et leur polarité mais aussi d'augmenter la protection du AH contre la dégradation par les glycanase et maintenir et/ou prolonger encore l'effet favorable des RGTA et AH sur le microenvironnement cellulaire.

En outre, cet exemple démontre clairement que des exemples de composition selon l'invention comprenant un polymère de formule AaXxYy ou AaXxYyZz et de l'acide hyaluronique permet avantageusement d'améliorer l'aspect de la peau, permet avantageusement d'améliorer la structure de la peau, notamment de part une augmentation de la sécrétion des collagènes et leur polarité mais aussi d'augmenter la protection du AH contre la dégradation par les glycanases. En outre, cet exemple démontre clairement que des exemples de composition selon l'invention comprenant un polymère de formule AaXxYy ou AaXxYyZz et de l'acide hyaluronique permet avantageusement d'obtenir un effet durable dans le temps et/ou permet avantageusement de prolonger encore l'effet synergique favorable des polymères de formule AaXxYy ou AaXxYyZz et de l'acide hyaluronique sur le microenvironnement cellulaire.

### Exemple 7 : Utilisation de polymères de formule AaXxYy ou AaXxYyZz et de l'acide hyaluronique dans le traitement de la sécheresse vaginale par injection

Dans cet exemple la composition utilisée est la composition décrite dans l'exemple 5 ci-dessus comprenant 10 µg/mL de OTR4120 et 20mg/mL de AH de différentes sources dans du sérum physiologique. Le mélange se fait en prélevant 100 µL d'une solution de OTR4120 à l'aide d'une seringue, puis après avoir relié par un adaptateur cette seringue à celle contenant la solution de AH à injecter, faire passer d'une seringue à l'autre les contenus.

Dans un cas des petites injections, à savoir 100 µl de composition, très superficielles du produit de l'invention ont été réalisées dans les muqueuses de la zone située entre le vagin et l'ouverture des petites lèvres. Elles sont réparties sur une dizaine de sites autour de la circonférence du vagin, juste derrière l'ouverture. Les zones d'injection ont été ensuite massées après, pour étaler le produit uniformément. Une comparaison de l'effet de comblement et/ou hydratation du tissu entre la composition comprenant un polymère de formule AaXxYy ou AaXxYyZz et de l'acide hyaluronique par rapport à une composition usuelle comprenant unique de l'acide hyaluronique est effectuée. Selon le médecin, l'effet obtenu avec un exemple de composition selon l'invention, à savoir comprenant un polymère de formule AaXxYy ou AaXxYyZz et de l'acide hyaluronique a été considéré comme meilleure sur la durée que le traitement avec une composition comprenant uniquement de l'acide hyaluronique.

Cet exemple démontre clairement que des exemples de composition selon l'invention comprenant un polymère de formule AaXxYy ou AaXxYyZz et de l'acide hyaluronique permet avantageusement de traiter et/ou d'améliorer la sécheresse vaginale. En particulier, cet exemple démontre clairement que des exemples de composition selon l'invention permet d'hydrater des tissus biologiques, en particulier des muqueuses, notamment la muqueuse vaginale.

### Exemple 8 : Utilisation de polymères de formule AaXxYy ou AaXxYyZz et de l'acide hyaluronique pour le traitement de sécheresse vaginale

Dans cet exemple la composition utilisée est la composition décrite dans l'exemple 7 ci-dessus à savoir une composition contenant 10 µg/mL de OTR4120 et 20mg/mL de AH de différentes sources dans du sérum physiologique. Le gel est déposé sur le doigt et un massage des muqueuses vaginal est réalisé. Ce traitement peut se faire quelques semaines après injection réalisée selon l'exemple 7.

L'utilisation d'un gel réalisé avec de l'OTR41203 a également été reportée comme très efficace.

Cet exemple démontre clairement que des exemples de composition selon l'invention comprenant un polymère de formule AaXxYy ou AaXxYyZz et de l'acide hyaluronique permet avantageusement de traiter et/ou d'améliorer la sécheresse vaginale. En particulier, cet exemple démontre clairement que des exemples de composition selon l'invention, notamment en application topique, permet d'hydrater des tissus biologiques, en particulier des muqueuses, notamment la muqueuse vaginale.

### Exemple 9 : Utilisation de polymères de formule AaXxYy ou AaXxYyZz et de l'acide hyaluronique pour le traitement de la sécheresse oculaire.

Dans cet exemple la composition utilisée correspond à un collyre comprenant de l'acide hyaluronique dans lequel du CACICOL (marque de commerce) à base de RGTA OTR4120 à 100 µg/mL. Ainsi, 3 à 4 dosettes de CACICOL soit environ 1,3 mL d'OTR4120 à 100µg/mL ou 1 mL de CACIPLIQ (OTR4120 à 100 µg/mL) sont ajoutés à 10 mL d'une solution à 0,15g/mL d'Acide Hyaluronique (par exemple dans un flacon multidose de type ABAK de théalose (laboratoires Théa) ou Viskyal ou Aqualarm.

Plusieurs patients atteints de sécheresse oculaire sévère (Sjogren) ont été traité avec le mélange collyre comprenant de l'acide hyaluronique et un polymère de formule AaXxYy ou AaXxYyZz, à savoir l'OTR4120, par administration d'une goutte toutes les 6 heures. Très rapidement le besoin d'ajouter la goutte du mélange avec cette fréquence s'estompe pour arriver à 2 puis 1 goutte par jour et se stabiliser à une goutte tous les deux ou trois jours. Une diminution de la sécheresse oculaire a été observée notamment par une diminution du besoin du patient d'appliquer le produit et/ou de la diminution de la fréquence du traitement et de la durée du traitement, notamment par rapport au collyre usuel. Cet effet est décrit pour le collyre CACICOL (Evaluation of a new matrix regenerating agent in patients with Sjögren syndrome and superficial ulcerative keratitis resistant to conventional therapy: A report of 3 cases. Fajnkuchen F, Barritault D, Giocanti-Aurégan A. Medicine (Baltimore). 2018 Mar;97(10):e9935. doi: 10.1097/MD.0000000000009935. [29]). Toutefois et de manière surprenant l'effet est beaucoup plus rapide avec la combinaison selon l'invention à savoir un polymère de formule AaXxYy ou AaXxYyZz et de l'acide hyaluronique, et en outre plus stable, permettant notamment avantageusement de favoriser la reconstruction d'un système lacrymal plus pérenne, en particulier avec un maintien d'application de la composition selon l'invention.

Cet exemple démontre clairement que des exemples de composition selon l'invention comprenant un polymère de formule AaXxYy ou AaXxYyZz et de l'acide hyaluronique permet avantageusement de traiter et/ou d'améliorer la sécheresse oculaire. En particulier, cet exemple démontre clairement que des exemples de composition selon l'invention, notamment en application topique, permet d'hydrater des tissus biologiques, en particulier la cornée, et permet avantageusement de favoriser et/ou restaurer le système lacrymal.

### Exemple 10 : Utilisation de polymères de formule AaXxYy ou AaXxYyZz et de l'acide hyaluronique pour réhydratation, préservation de la sécheresse et repousse des cheveux

Dans cet exemple le produit utilisé est constitué de la crème laluset (laboratoire Genevrier (www.laboratoires-genevrier.com) contenant un mélange de 0,2% d'acide Hyaluronique dans 100g mélangé à 1mg de OTR41302 dans du sérum physiologique. Cette crème est utilisée tous les 3 jours en massage des cheveux.

Ce traitement montre un effet de prévention de la perte des cheveux, pouvant avantageusement chez des sujets traités stimuler la repousse de cheveux alors que des compositions contenant uniquement de l'AH seul ou du RGTA seul n'ont pas montrés d'effets, en particulier aucun effet remarquable.

La composition décrite dans l'exemple 8 ci-dessus est également utile pour cette application.

Cet exemple démontre clairement que des exemples de composition selon l'invention comprenant un polymère de formule AaXxYy ou AaXxYyZz et de l'acide hyaluronique permet avantageusement de réhydrater le cuir chevelu, tout en hydratant la fibre capillaire. En outre, cet exemple démontre clairement que des exemples de composition selon l'invention comprenant un polymère de formule AaXxYy ou AaXxYyZz et de l'acide hyaluronique permet avantageusement de prévenir la chute de cheveux et/ou de stimuler la repousse capillaire. En outre, cet exemple démontre clairement que des exemples de composition selon l'invention comprenant un polymère de formule AaXxYy ou AaXxYyZz et de l'acide hyaluronique permet avantageusement de prévenir et/ou traiter l'alopécie.

### Exemple 11 : Utilisation de polymères de formule AaXxYy ou AaXxYyZz et de l'acide hyaluronique pour le traitement des brulures superficielles de la peau (thermique ou post radique) ou des lésions induites par le peeling et des ulcères de peau par décollement de l'épiderme ou des lésions de la peau ou des muqueuses par exemple après une épisiotomie.

Dans cet exemple, les composés utilisés sont identiques à ceux décrits dans l'exemple 6 ou celui décrit dans l'exemple 10 ci-dessus.

Des compositions sous une forme de spray comprenant 0,05 à 1% d'acide hyaluronique et 10µg/mL de OTR4132 ou OTR4135 ou OTR4120 ou OTR4122 ou OTR4125 dans sérum physiologique (ou de l'eau) sont préparées. Ces compositions se sont avérées particulièrement judicieuses et efficaces pour traiter des brulures de surface. En outre, l'utilisation de la formulation, notamment sous la forme de spray, a permis d'éviter la douleur et le risque d'aggravation de la lésion susceptible d'intervenir lors de l'application de la composition.

Avantageusement, cette formule de solution aqueuse pour spray ne contient pas d'acide gras ce qui évite le risque d'induire par la présence de résidus d'acides gras encore non éliminée et provenant de crème sont la cause d'une aggravation de la brulure par un effet « barbecue »

Cette formulation a montré également une efficacité, de manière synergique, à induire une cicatrisation de meilleure qualité et à réduire la douleur qu'aucun des produits utilisés séparément ne permettait d'observer.

Les produits à base des RGTA de très hauts poids moléculaires, notamment OTR4122, OTR4125 et les formes contenant des groupements acétylés d'OTR4132, OTR4135 dispensés en spray et en mélange avec de l'acide hyaluronique ce sont montrés encore plus efficaces, notamment lorsque ce spray a été utilisé sur des patients atteints de l'épidermolyse bulleuse améliorant encore les effets décrits dans le cas de cette pathologie avec l'OTR4120 seul (A Rapid Response to Matrix Therapy With RGTA in Severe Epidermolysis Bullosa. Malaq AA, Barritault D. Eplasty. 2012;12:ic15 [30]). En outre, l'utilisation de la combinaison et/ou composition selon l'invention permet avantageusement l'obtention des plaies fermées plus solide et/ou dont la cicatrisation est plus efficace, par exemple les plaies fermées apparaissent plus résistantes à la réouverture.

De même la douleur a été remarquablement réduite lors de l'application topique du produit sous forme de spray ou sous forme de gel comme décrit dans l'exemple sur l'épidermolyse bulleuse ou lors de la cicatrisation d'une épisiotomie par application topique du gel (selon l'exemple 8) ou du spray

Ces exemples démontrent clairement que des exemples de composition selon l'invention comprenant un polymère de formule AaXxYy ou AaXxYyZz et de l'acide hyaluronique permet avantageusement favoriser la cicatrisation, par exemple de plaies et/ou de brulures cutanées. En outre, cet exemple démontre clairement que des exemples de composition selon l'invention comprenant un polymère de formule AaXxYy ou AaXxYyZz et de l'acide hyaluronique permet avantageusement de favoriser la cicatrisation d'ulcères cutanés issus de l'épidermolyse bulleuses, ou de lésion par incision ou déchirement des muqueuses et en outre avantageusement permet une cicatrisation durable avec une qualité cicatricielle augmentée et une réduction de la douleur.

### Exemple 12 : Utilisation de polymères de formule AaXxYy ou AaXxYyZz et de l'acide hyaluronique dans les traitements des aphtes, gingivites, ou mucites

Dans cet exemple, des formulations aqueuses comprenant du RGTA OTR4120 ou RGTA OTR41202 à 10 µg/mL mélangé avec de l'acide hyaluronique (10mg/mL) sont préparées. Les solutions aqueuses sont indépendamment disposés dans des flacons en verre de 20 mL fermé par un bouchon muni d'une pompe délivrant 140 µL/ à chaque pression. La composition est aspergée directement dans la bouche des patients ayant des aphtes, gingivites, ou mucites. Ainsi les produits de l'invention, notamment administrés sous forme de spray, montrent une efficacité bien meilleure sur le traitement des aphtes, gingivites, et mucites et en particulier l'effet sur le traitement des aphtes, gingivites, et mucites est plus rapide que chacun des produits pris séparément.

Cet exemple démontre clairement que des exemples de composition selon l'invention comprenant un polymère de formule AaXxYy ou AaXxYyZz et de l'acide hyaluronique permet avantageusement de traiter des plaies et/ou pathologie buccales. En particulier, cet exemple démontre clairement que des exemples de composition selon l'invention comprenant un polymère de formule AaXxYy ou AaXxYyZz et de l'acide hyaluronique permet avantageusement de traiter des aphtes, gingivites, et mucites et de favoriser ainsi la réparation et/ou d'améliorer l'aspect et/ou la structure des muqueuses buccales.

### Exemple 13 : Utilisation de polymères de formule AaXxYy ou AaXxYyZz et de l'acide hyaluronique dans le traitement des altérations du système locomoteur (articulation, tendons, ligaments, discs intervertébraux) et articulation mandibulaires

Dans cet exemple les formulations utilisées combinent le RGTA OTR4120 ou OTR4131 à 10 µg/mL avec de l'acide hyaluronique habituellement utilisé à des concentrations préférées de 10 à 25 mg/mL comme celles utilisées dans l'exemple 5.

La composition est appliquée par injection à proximité de la zone lésée ou directement sur le site de la lésion par exemple par voie intra articulaire si la lésion est chondrale, ostéoarticulaire ou intra tendineuse ou péri tendineuse ou intra musculaire dans le cas de déchirure ou de claquage. Les produits de l'invention diffusant facilement dans et autour de la zone de lésion remplissant l'espace créé par la lésion.

Les personnes traitées présentent indépendamment une pathologie et/ou altération du système locomoteur, par exemple au niveau des articulations, à savoir comme dans l'arthrite du genoux ou de la hanche ou des troubles temporaux mandibulaire, au niveau des tendons, à savoir ruptures partielles des fibres tendineuses ou ligamentaires comme déjà décrit par Jacquet-Guibon S, 2018 [20] avec les produits OTR4131 chez le cheval de sport ou dans la revue Barritault D et al. [18] notamment dans la fusion de vertèbre [18] ou injection proximale au niveau de la zone intra discal ou dans la thèse de Carnicer David, Preliminary report-ultrasonographic evolution of tendon lesions treated with RGTA in horses, Ecole Nationale Vétérinaire de Maison Alfort , Février 2009 [36], au niveau des ligaments dans un exemple de troubles de l'articulation mandibulaire traitée par injection dans la zone au niveau péri-articulaire à proximité du disque articulaire fibro-cartilagineux, des ligaments discaux et des muscles manducateurs ou encore au niveau des discs intervertébraux, à savoir de la zone d'aplatissement d'un disc intervertébral (L1).

Dans ces traitements le résultat et effet est rapide, notamment une disparition immédiate de la douleur, et en outre avantageusement l'effet est de longue durée, sur plus d'une année, notamment en association avec une application topique d'un exemple de composition selon l'invention, notamment de la crème décrite dans l'exemple 6 comprenant avantageusement des polymères de petits poids moléculaires, par exemple de 3000 à 6000 daltons associés à des AH ou pas.

Avantageusement, les résultats obtenus et les effets avec un exemple de composition selon l'invention sont supérieurs à ceux obtenus avec l'utilisation de RGTA seul tel que décrit dans l'étude Jacquet-Guibon S, 2018 [20] ou Carnicer [36]

En outre, avantageusement effet particulièrement bénéfique, surprenant et inattendu a été observé pour une préparation injectable pour les traitements des lésions articulaires et tendineuses. Il en a été de même pour un cas de douleur liés à des lésions de disques intervertébraux, la douleur ayant disparue sans réapparaitre après plus d'une année.

Cet exemple démontre clairement que des exemples de composition selon l'invention comprenant un polymère de formule AaXxYy ou AaXxYyZz et de l'acide hyaluronique permet avantageusement de traiter des pathologies et/ou conditions du système locomoteur. En particulier, cet exemple démontre clairement que des exemples de composition selon l'invention comprenant un polymère de formule AaXxYy ou AaXxYyZz et de l'acide hyaluronique permet avantageusement de traiter des lésions articulaires, tendineuses, des pathologies liées au disque intervertébraux, par exemple un tassement du disque, une lésion du disque, par exemple en restaurant sa structure et/ou en favorisant sa cicatrisation. En outre cet exemple démontre également clairement que des exemples de composition selon l'invention comprenant un polymère de formule AaXxYy ou AaXxYyZz et de l'acide hyaluronique permet avantageusement de traiter de manière synergique les douleurs, par exemple les douleurs liées à des lésions de disques intervertébraux.

### Exemple 14 : Utilisation de polymères de formule AaXxYy ou AaXxYyZz et de l'acide hyaluronique dans le traitement de maladies parodontales

Dans cet exemple, la composition utilisée est indépendamment celle décrite dans les exemples 5 ou 13. La composition est utilisée indépendamment notamment en application locales au niveau de la lésion par remplissage, injection des poches parodontales, greffes osseuses.

La composition est, soit directement injectée dans les gencives ou dans la zone de la poche parodontale, soit déposée dans cette poche.

Après application, la composition selon l'invention a permis une meilleure résorption des lésions parodontales ou prise de greffe qu'observés avec les produits à base du polymère formule AaXxYy ou AaXxYyZz utilisés seul.

En outre, avantageusement, un exemple de composition selon l'invention a permis avantageusement un traitement des pathologies parodontales, notamment un comblement du déficit de tissus de soutient des dents et une restauration des gencives.

En outre, les résultats obtenus pas un exemple de composition selon l'invention montrent une meilleure efficacité et, de manière inattendue, un effet synergique dans le traitement par rapport à des résultats obtenus avec le RGTA seul revue, par exemple tels que décrits dans Barritault D et al. 2017 [18] ou, de l'avis de l'homme de l'art, par rapport à des résultats obtenus avec le AH seul.

### Exemple 15- pate dentaire comprenant des polymères de formule AaXxYy ou AaXxYyZz et de l'acide hyaluronique

La composition selon l'invention comprenant un polymère biocompatible et de l'acide hyaluronique peut être incorporé facilement dans toutes sortes de pates dentaires et utilisé pour une application topique comme dentifrice.

Dans cet exemple, les pates dentaires sont des pâtes, notamment pour utilisation biquotidienne comprenant indépendamment une concentration de RGTA OTR4120 ou OTR4131 de 1 µg/mL et une concentration d'acide hyaluronique de 20 mg/mL.

La pâte dentaire est utile, notamment, pour une utilisation biquotidienne et permet avantageusement le traitement et/ou la prévention de maladies parodontales.

### Exemple 16 : Utilisation de polymères de formule AaXxYy ou AaXxYyZz et de l'acide hyaluronique dans le traitement de la fibrose, exemple des adhérences tissulaires

Dans cet exemple, des compositions comprenant une concentration de RGTA OTR4120 ou OTR4131 de 10 µg/mL et une concentration d'acide hyaluronique de 5 à 20 mg/mL est utilisée.

Comme démontré dans l'exemple 2, le RGTAOTR4120 ou OTR4131 à 10µg/mL combiné à l'Acide Hyaluronique a un effet synergique non seulement pour diminuer la quantité de Collagène 3 surexprimée dans la fibrose que d'augmenter celle du collagène 1 de manière à obtenir un rapport COL3/COL1 très proche de celui d'un tissu normal mais également par sa capacité favoriser la sécrétion du coté basal et non apical.

Cette propriété du mélange est notamment mise en valeur dans une utilisation du produit de l'invention sous forme de spray, par exemple comme décrit dans les exemples 6, 10, 12, pour une application post chirurgie ouverte. Par exemple après laparotomie en couvrant les tissus et organes par une pulvérisation du spray avant fermeture, ou au moment d'introduction d'un implant, par exemple un pace maker, prothèses, cathéters dont on veut réduire l'adhérence du tissu conjonctif qui se fait au cours du temps.

Dans ces indications l'utilisation du spray s'est montrée très efficace et a pu être vérifiée notamment chez un patient pour lequel une deuxième laparotomie a été effectuée.

Cet exemple démontre clairement que des exemples de composition selon l'invention comprenant un polymère de formule AaXxYy ou AaXxYyZz et de l'acide hyaluronique permet avantageusement de prévenir et/ou traiter les fibroses. En particulier, cet exemple démontre clairement que des exemples de composition selon l'invention comprenant un polymère de formule AaXxYy ou AaXxYyZz et de l'acide hyaluronique permet avantageusement de prévenir la formation de plaque d'adhérence, par exemple au niveau abdominal, par exemple après chirurgie abdominale.

### Exemple 17 : Utilisation de polymères de formule AaXxYy ou AaXxYyZz et de l'acide hyaluronique dans le traitement de la fibrose

Dans cet exemple, des compositions comprenant une concentration de RGTA OTR4120 ou OTR4131 de 10 µg/mL et une concentration d'acide hyaluronique de 0,1 à 2 mg/mL est utilisée.

Dans cet exemple, les fibroses sont des fibroses notamment présentes au niveau du muscle ou du cerveau ischémié.

L'administration des compositions est effectuée selon les procédés usuels connus de l'homme du métier.

Le RGTA est connu pour des effets protecteurs et antifibrotiques sur la récupération fonctionnelle du muscle [26 ; 27] ou du cerveau ischémié (Theranostics. 2018 Nov 12;8(21):5814-5827. doi: 10.7150/thno.28252. eCollection 2018., A heparan sulfate-based matrix therapy reduces brain damage and enhances functional recovery following stroke. Khelif Y, Toutain J, Quittet MS, Chantepie S, Laffray X, Valable S, Divoux D, Sineriz F, Pascolo-Rebouillat E, Papy-Garcia D, Barritault D, Touzani O, Bernaudin M. [31]).

Dans cet exemple, la combinaison de RGTA, par exemple OTR4120 ou 4131 avec de l'acide hyaluronique aura également un effet synergique dans le traitement de la fibrose, et en outre de la récupération fonctionnelle du muscle fibrotique traité.

Cet exemple démontre clairement que des exemples de composition selon l'invention comprenant un polymère de formule AaXxYy ou AaXxYyZz et de l'acide hyaluronique peut permettre avantageusement de traiter les fibroses, notamment des fibroses musculaires. En particulier, cet exemple démontre clairement que des exemples de composition selon l'invention comprenant un polymère de formule AaXxYy ou AaXxYyZz et de l'acide hyaluronique peut permettre avantageusement de prévenir la formation de fibrose tout en améliorant la récupération fonctionnelle du tissu, par exemple du muscle fibrotique.

### Exemple 18 : Utilisation de polymères de formule AaXxYy ou AaXxYyZz et de l'acide hyaluronique dans le traitement des maladies dégénératives.

Dans cet exemple, des compositions comprenant une concentration de RGTA OTR4120 ou OTR4131 de 10 µg/mL et une concentration d'acide hyaluronique de 0,1 à 2 mg/mL est utilisée.

Le RGTA est également connu pour ses effets dans le traitement des maladies neurodégénératives (EP 12305414 [32]), notamment par administration intra-artérielle ou intracérébrale du RGTA.

L'administration des compositions est effectuée selon les procédés usuels connus de l'homme du métier, notamment par administration intra-artérielle ou intracérébrale.

Les sujets présentent notamment des maladies neurodégénératives, par exemple tel que décrit dans le document EP 12305414 [32].

Dans cet exemple, la combinaison de RGTA, par exemple OTR4120 ou 4131 avec de l'acide hyaluronique aura également un effet synergique dans le traitement des maladies neurodégénératives.

### Liste de références

1. Modified hyaluronic acid based materials for biomedical applications. Tiwari S, Bahadur P.Int J Biol Macromol. 2019 Jan;121:556-571.doi: 10.1016/j.ijbiomac.2018.10.049. Epub 2018 Oct 12. Review.
2. Hyaluronic acid, a promising skin rejuvenating biomedicine: A review of recent updates and pre-clinical and clinical investigations on cosmetic and nutricosmetic effects. Bukhari SNA, Roswandi NL, Waqas M, Habib H, Hussain F, Khan S, Sohail M, Ramli NA, Thu HE, Hussain Z. Int J Biol Macromol. 2018 Dec;120(Pt B):1682-1695. doi: 10.1016/j.ijbiomac.2018.09.188. doi:10.1016/j.ijbiomac.2018.09.188
3. Reduction of postoperative adhesion development. Diamond MP. Fertil Steril. 2016 Oct;106(5):994-997.e1. doi: 10.1016/j.fertnstert.2016.08.029. Epub 2016 Sep 10. Review.
4. Document brevet US06689741, US2014301972A1
5. Reversal of abnormal collagen production in Crohn's disease intestinal biopsies treated with regenerating agents. Alexakis C, Caruelle JP, Sezeur A, Cosnes J, Gendre JP, Mosnier H, Beaugerie L, Gallot D, Malafosse M, Barritault D, Kern P. Gut. 2004 Jan;53(1):85-90.
6. Insights on a new path of pre-mitochondrial apoptosis regulation by a glycosaminoglycan mimetic. Yue XL, Lehri S, Li P, Barbier-Chassefière V, Petit E, Huang QF, Albanese P, Barritault D, Caruelle JP, Papy-Garcia D, Morin C. Cell Death Differ. 2009 May;16(5):770-81. doi: 10.1038/cdd.2009.9
7. Differential effect triggered by a heparan mimetic of the RGTA family preventing oral mucositis without tumor protection. Mangoni M, Yue X, Morin C, Violot D, Frascogna V, Tao Y, Opolon P, Castaing M, Auperin A, Biti G, Barritault D, Vozenin-Brotons MC, Deutsch E, Bourhis J. Int J Radiat Oncol Biol Phys. 2009 Jul 15;74(4):1242-50. doi: 10.1016/j.ijrobp.2009.03.006
8. RGTA OTR4120, a heparan sulfate mimetic, is a possible long-term active agent to heal burned skin. Garcia-Filipe S, Barbier-Chassefiere V, Alexakis C, Huet E, Ledoux D, Kerros ME, Petit E, Barritault D, Caruelle JP, Kern P J Biomed Mater Res A. 2007 Jan;80(1):75-8
9. Document brevet EP1677807
10. Heparin-like synthetic polymers, named RGTAs, mimic biological effects of heparin in vitro. Rouet V, Meddahi-Pellé A, Miao HQ, Vlodavsky I, Caruelle JP, Barritault D. J Biomed Mater Res A. 2006 Sep 15;78(4):792-7
11.FGF protection and inhibition of human neutrophil elastase by carboxymethyl benzylamide sulfonate dextran derivatives. Meddahi A, Lemdjabar H, Caruelle JP, Barritault D, Hornebeck W. Int J Biol Macromol. 1996 Feb;18(1-2):141-5.
12. Human plasmin enzymatic activity is inhibited by chemically modified dextrans. Ledoux D, Papy-Garcia D, Escartin Q, Sagot MA, Cao Y, Barritault D, Courtois J, Hornebeck W, Caruelle JP. J Biol Chem. 2000 Sep 22;275(38):29383-90
13. Heparan sulfate mimetics modulate calpain activity during rat Soleus muscle regeneration. Zimowska M, Szczepankowska D, Streminska W, Papy D, Tournaire MC, Gautron J, Barritault D, Moraczewski J, Martelly I. J Cell Physiol. 2001 Aug;188(2):178-87.
14. RGTA® or ReGeneraTing Agents mimic heparan sulfate in regenerative medicine: from concept to curing patients. Barritault D, Gilbert-Sirieix M, Rice KL, Siñeriz F, Papy-Garcia D, Baudouin C, Desgranges P, Zakine G, Saffar JL, van Neck J. Glycoconj J. 2017 Jun;34(3):325-338. doi: 10.1007/s10719-016-9744-5
15.Tammi R., Agren UM., Tuhkanen AL., Tammi M. Hyaluronan metabolism in skin. Progress in Histochemistry & Cytochemistry. 29(2):1-81, 1994
16.R. Stern et al., European Journal of Cell Biology 58 (2006) 699-715)
17.Yasunori I. et al., Biomaterials 2011, 32 :769e776) et Petit E. et al,. Biomacromolecules. 2004 Mar-Apr; 5(2):445-52.
18. RGTA®-based matrix therapy - A new branch of regenerative medicine in locomotion. Barritault D, Desgranges P, Meddahi-Pellé A, Denoix JM, Saffar JL. Joint Bone Spine. 2017 May;84(3):283-292. doi: 10.1016/j.jbspin.2016.06.012.
19.Frescaline G. et al., Tissue Eng Part A. 2013 Jul;19(13-14):1641-53. doi: 10.1089/ten.TEA.2012.0377).
20. Randomized controlled trial demonstrates the benefit of RGTA® based matrix therapy to treat tendinopathies in racing horses. Jacquet-Guibon S, Dupays AG, Coudry V, Crevier-Denoix N, Leroy S, Siñeriz F, Chiappini F, Barritault D, Denoix JM. PLoS One. 2018 Mar 9:13(3):e0191796. doi: 10.1371/journal.pone.0191796
21. Heparan sulfate proteoglycans mediate internalization and propagation of specific proteopathic seeds. Holmes BB, DeVos SL, Kfoury N, Li M, Jacks R, Yanamandra K, Ouidja MO, Brodsky FM, Marasa J, Bagchi DP, Kotzbauer PT, Miller TM, Papy-Garcia D, Diamond MI. Proc Natl Acad Sci U S A. 2013 Aug 13;110(33):E3138-47. doi: 10.1073/pnas.1301440110
22. Structure-activity studies of heparan mimetic polyanions for anti-prion therapies. Ouidja MO, Petit E, Kerros ME, Ikeda Y, Morin C, Carpentier G, Barritault D, Brugère-Picoux J, Deslys JP, Adjou K, Papy-Garcia D. Biochem Biophys Res Commun. 2007 Nov 9;363(1):95-100.
23.In Vitro Evaluation of the Sensitivity of a Hyaluronic Acid PEG Cross-Linked to Bovine Testes Hyaluronidase Nicola Zerbinati et al Open Access Maced J Med Sci. 2018 Jan 25; 6(1):20-24 https://doi.org/10.3889/oamjms.2018
24. Sall I, Férard G. Comparison of the sensitivity of 11 crosslinked hyaluronic acid gels to bovine testis hyaluronidase. PolymDegrad Stab. 2007; 92: 915-919 . https://doi.org/10.1016/j.polymdegradstab.2006.11.020.
25.Reissig JL, Strominger JL, Leloir LF. A modified colorimetric method for the estimation of N-acetylamino sugars. J Biol Chem 1955;217:959e96.
26.A substituted dextran enhances muscle fiber survival and regeneration in ischemic and denervated rat EDL muscle. Desgranges P, Barbaud C, Caruelle JP, Barritault D, Gautron J. FASEB J. 1999 Apr;13(6):761-6.
27. New agents for the treatment of infarcted myocardium. Yamauchi H, Desgranges P, Lecerf L, Papy-Garcia D, Tournaire MC, Moczar M, Loisance D, Barritault D. FASEB J. 2000 Nov;14(14):2133-4.
28.Asselot-Chapel C, et al Expression of fibronectin and interstitial collagen genes in smooth muscle cells: modulation by low molecular weight heparin fragments and serum.Biochem Pharmacol. 1995 Mar 1;49(5):653-9.
29. Evaluation of a new matrix regenerating agent in patients with Sjögren syndrome and superficial ulcerative keratitis resistant to conventional therapy: A report of 3 cases. Fajnkuchen F, Barritault D, Giocanti-Aurégan A. Medicine (Baltimore). 2018 Mar;97(10):e9935. doi: 10.1097/MD.0000000000009935.
30.A Rapid Response to Matrix Therapy With RGTA in Severe Epidermolysis Bullosa. Malaq AA, Barritault D. Eplasty. 2012;12:ic15.
31.Theranostics. 2018 Nov 12;8(21):5814-5827. doi: 10.7150/thno.28252. eCollection 2018., A heparan sulfate-based matrix therapy reduces brain damage and enhances functional recovery following stroke. Khelif Y, Toutain J, Quittet MS, Chantepie S, Laffray X, Valable S, Divoux D, Sineriz F, Pascolo-Rebouillat E, Papy-Garcia D, Barritault D, Touzani O, Bernaudin M.
32.EP 12305414
33. Jammed Microgel Inks for 3D Printing Applications., Highley CB, Song KH, Daly AC, Burdick JA. Adv Sci (Weinh). 2018 Oct 24;6(1):1801076. doi: 10.1002/advs.201801076.
34. Collagen/heparin sulfate scaffolds fabricated by a 3D bioprinter improved mechanical properties and neurological function after spinal cord injury in rats. Chen C, Zhao ML, Zhang RK, Lu G, Zhao CY, Fu F, Sun HT, Zhang S, Tu Y, Li XH. J Biomed Mater Res A. 2017 May;105(5):1324-1332. doi: 10.1002/jbm.a.36011. Epub 2017
35. Development and Evaluation of Hyaluronic Acid-Based Hybrid Bio-Ink for Tissue Regeneration. Lee J, Lee SH, Kim BS, Cho YS, Park Y.Tissue Eng Regen Med. 2018 Sep 21;15(6):761-769. doi: 10.1007/s13770-018-0144-8. eCollection 2018 Dec.
36. Carnicer David , Preliminary report- ultrasonographic evolution of tendon lesions treated with RGTA in horses, Ecole Nationale Vétérinaire de Maison Alfort , Février 2009

## Revendications

1. Composition pharmaceutique pour son utilisation comme médicament pour le traitement de lésions du système respiratoire, de préférence de lésions pulmonaires, ladite composition comprenant
- un polymère biocompatible de formule générale (I) suivante
AaXxYy (I)
dans laquelle :
A représente un monomère,
X représente un groupement R₁COOR₂ ou -R₉(C=O)R₁₀,
Y représente un groupement O ou N-sulfonate et répondant à l'une des
formules suivante -R₃OSO₃R₄, -R₅NSO₃R₆, R₇SO₃R₈ dans lesquelles :
R₁, R₃, R₅ et R₉ représentent indépendamment une chaîne hydrocarbonée aliphatique, éventuellement ramifiée et/ou insaturée et qui contient éventuellement un ou plusieurs cycles aromatiques à l'exception de la benzylamine et de la benzylamine sulfonate, R₂, R₄, R₆ et R₈ représentent indépendamment un atome d'hydrogène ou un cation,
R₇ et R₁₀ représente indépendamment une liaison, une chaîne hydrocarbonée aliphatique, éventuellement ramifiée et/ou insaturée,
a représente le nombre de monomères,
x représente le taux de substitution des monomères A par des groupements X,
y représente le taux de substitution des monomères A par des groupements Y,
et
- de l'acide hyaluronique.

2. Composition pour son utilisation selon la revendication 1, dans laquelle les monomères A identiques ou différents sont choisis parmi les sucres, les esters, les alcools, les acides aminés, les nucléotides, les acides nucléiques, les protéines ou des dérivés de ceux-ci.

3. Composition pour son utilisation selon la revendication 1 ou 2, dans laquelle les monomères A identiques ou différents sont choisis parmi les sucres ou dérivés de ceux-ci.

4. Composition pour utilisation selon l'une quelconque des revendications 1 à 3, dans laquelle un maximum de 20% des monomères A sont indépendamment des monomères de formule suivante : 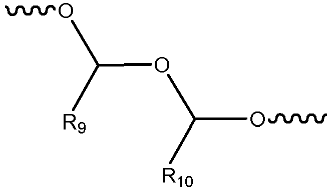 dans laquelle R₉ et R₁₀ représente indépendamment un atome d'oxygène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué, un groupement de formule -R₁₁-(X)ₙ-R₁₂ dans laquelle R₁₁ représente une chaine carbonée aliphatique en C₁ à C₄, éventuellement ramifiée et/ou insaturée, X représente un hétéroatome choisi parmi l'oxygène et l'azote, n est un entier compris de 1 à 4 et R₁₂ est un atome d'hydrogène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué.

5. Composition pour son utilisation selon l'une quelconque des revendications 1 à 4, dans laquelle le nombre de monomère « a » est tel que la masse desdits polymères de formule (I) est supérieure ou égale à 3000 daltons.

6. Composition pour son utilisation selon l'une quelconque des revendications 1 à 5, dans laquelle le taux de substitution « x » est compris entre 10 et 150%.

7. Composition pour utilisation selon l'une quelconque des revendications précédentes, dans laquelle le taux de substitution « y » est compris entre 10 et 170%.

8. Composition pour son utilisation selon l'une quelconque des revendications précédentes, dans laquelle que ledit polymère biocompatible comprend en outre des groupements chimiques fonctionnels Z, différents de X et Y, capables de conférer audit polymère des propriétés biologiques ou physicochimiques supplémentaires.

9. Composition pour son utilisation selon la revendication 8, dans laquelle le taux de substitution « z » de l'ensemble des monomères A par des groupements Z est compris de 1 à 50%.

10. Composition pour son utilisation selon la revendication 8 ou 9, dans laquelle le groupement Z est une substance capable de conférer auxdits polymères une meilleure solubilité ou lipophilie.

11. Composition pour son utilisation selon la revendication 10, dans laquelle les groupements Z sont identiques ou différents et sont choisis dans le groupe comprenant des acides aminés, des acides gras, des alcools gras, des céramides, ou des dérivés de ceux-ci, ou encore des séquences nucléotidiques d'adressage.

12. Composition pour son utilisation selon l'une quelconque des revendications 4 à 11 dans laquelle les groupements R₉ et R₁₀ sont indépendamment et éventuellement substitués par un groupement Z.

## Patentansprüche

1. Pharmazeutische Zusammensetzung zur Verwendung als Medikament zur Behandlung von Läsionen des Atmungssystems, vorzugsweise von Lungenläsionen, wobei die Zusammensetzung umfasst
- ein biokompatibles Polymer der folgenden allgemeinen Formel (I)
AaXxY (I)
in dem:
A steht für ein Monomer,
X eine Gruppe R₁COOR₂ oder -R₉(C=O)R₁₀ darstellt,
Y eine O- oder N-Sulfonatgruppe darstellt und eine der folgenden Formeln
aufweist: -R₃OSO₃R₄, -R₅NSO₃R₍₆₎, R₇SO₃R₈, in denen:
R₁, R₃, R₅ und R₉ unabhängig voneinander eine aliphatische Kohlenwasserstoffkette darstellen, die gegebenenfalls verzweigt und/oder ungesättigt ist und die gegebenenfalls einen oder mehrere aromatische Ringe enthält, mit Ausnahme von Benzylamin und Benzylaminsulfonat, R₂, R₄, R₆ und R₈ unabhängig voneinander ein Wasserstoffatom oder ein Kation darstellen,
R₇ und R₁₀ stehen unabhängig voneinander für eine Bindung oder eine aliphatische Kohlenwasserstoffkette, gegebenenfalls verzweigt und/oder ungesättigt,
a steht für die Anzahl der Monomere,
x steht für die Substitutionsrate der Monomere A durch Gruppen X,
y die Substitutionsrate der Monomere A durch Gruppen Y darstellt und
- Hyaluronsäure.

2. Zusammensetzung zur Verwendung nach Anspruch 1, **dadurch gekennzeichnet, dass** die gleichen oder unterschiedlichen Monomere A ausgewählt sind aus Zuckern, Estern, Alkoholen, Aminosäuren, Nukleotiden, Nukleinsäuren, Proteinen oder Derivaten davon.

3. Zusammensetzung zur Verwendung nach Anspruch 1 oder 2, **dadurch gekennzeichnet, dass** die gleichen oder unterschiedlichen Monomere A aus Zuckern oder deren Derivaten ausgewählt sind.

4. Zusammensetzung zur Verwendung nach einem der Ansprüche 1 bis 3, wobei maximal 20 % der Monomere A unabhängig voneinander Monomere der folgenden Formel sind: 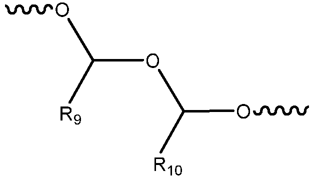 in der R₉ und R₁₀ unabhängig voneinander ein Sauerstoffatom, eine gegebenenfalls verzweigte und/oder ungesättigte aliphatische Kohlenwasserstoffkette, eine Heteroarylgruppe, die unabhängig voneinander ein oder mehrere Sauerstoff- und/oder Stickstoffatome enthält, eine Aldehydfunktion, eine Carbonsäuregruppe, ein Diol, ein substituiertes Diol, eine Gruppe der Formel -R₁₁-(X)ₙ-R₁₂, in der R₁₁ eine gegebenenfalls verzweigte und/oder ungesättigte aliphatische Kohlenstoffkette C₁ bis C₄ darstellt, bedeuten X ein Heteroatom, ausgewählt aus Sauerstoff und Stickstoff, n eine ganze Zahl von 1 bis 4 und R₁₂ ein Wasserstoffatom, eine gegebenenfalls verzweigte und/oder ungesättigte aliphatische Kohlenwasserstoffkette, eine Heteroarylgruppe, die unabhängig ein oder mehrere Sauerstoff- und/oder Stickstoffatome enthält, eine Aldehydfunktion, eine Carbonsäuregruppe, ein Diol oder ein substituiertes Diol bedeuten.

5. Zusammensetzung zur Verwendung nach einem der Ansprüche 1 bis 4, **dadurch gekennzeichnet, dass** die Anzahl der Monomere "a" so gewählt ist, dass die Masse der Polymere der Formel (I) größer oder gleich 3000 Dalton ist.

6. Zusammensetzung zur Verwendung nach einem der Ansprüche 1 bis 5, wobei der Substitutionsgrad "x" zwischen 10 und 150 % liegt.

7. Zusammensetzung zur Verwendung nach einem der vorhergehenden Ansprüche, wobei der Substitutionsgrad "y" zwischen 10 und 170 % liegt.

8. Zusammensetzung zur Verwendung nach einem der vorhergehenden Ansprüche, wobei das biokompatible Polymer weiterhin funktionelle chemische Gruppen Z umfasst, die sich von X und Y unterscheiden und in der Lage sind, dem Polymer zusätzliche biologische oder physikochemische Eigenschaften zu verleihen.

9. Zusammensetzung zur Verwendung nach Anspruch 8, **dadurch gekennzeichnet, dass** der Substitutionsgrad "z" aller Monomere A durch Gruppen Z zwischen 1 und 50 % liegt.

10. Zusammensetzung zur Verwendung nach Anspruch 8 oder 9, **dadurch gekennzeichnet, dass** die Gruppe Z eine Substanz ist, die geeignet ist, den genannten Polymeren eine verbesserte Löslichkeit oder Lipophilie zu verleihen.

11. Zusammensetzung zur Verwendung nach Anspruch 10, wobei die Z-Gruppen gleich oder verschieden sind und ausgewählt sind aus der Gruppe bestehend aus Aminosäuren, Fettsäuren, Fettalkoholen, Ceramiden oder Derivaten davon oder Nukleotid-Adressierungssequenzen.

12. Zusammensetzung zur Verwendung nach einem der Ansprüche 4 bis 11, wobei die Gruppen R₉ und R₁₀ unabhängig und gegebenenfalls mit einer Gruppe Z substituiert sind.

## Claims

1. A pharmaceutical composition for use as a medicament for the treatment of lesions of the respiratory system, preferably lung lesions, said composition comprising
- a biocompatible polymer of the following general formula (I)
AaXxY (I)
in which :
A represents a monomer,
X represents a group R₁COOR₂ or -R₉(C=O)R₁₀,
Y represents an O or N-sulphonate group and has one of the following
formulae -R₃OSO₃R₄, -R₅NSO₃R₆, R₇SO₃R₈ in which :
R₁, R₃, R₅ and R₉ independently represent an aliphatic hydrocarbon chain, optionally branched and/or unsaturated and which optionally contains one or more aromatic rings with the exception of benzylamine and benzylamine sulphonate, R₂, R₄, R₆ and R₈ independently represent a hydrogen atom or a cation,
R₇ and R₁₀ independently represent a bond or an aliphatic hydrocarbon chain, optionally branched and/or unsaturated,
a represents the number of monomers,
x represents the rate of substitution of monomers A by groups X,
y represents the rate of substitution of monomers A by groups Y, and
- hyaluronic acid.

2. Composition for use according to claim 1, wherein the identical or different monomers A are selected from sugars, esters, alcohols, amino acids, nucleotides, nucleic acids, proteins or derivatives thereof.

3. Composition for use according to claim 1 or 2, wherein the identical or different monomers A are selected from sugars or derivatives thereof.

4. A composition for use according to any one of claims 1 to 3, wherein a maximum of 20% of the monomers A are independently monomers of the following formula : 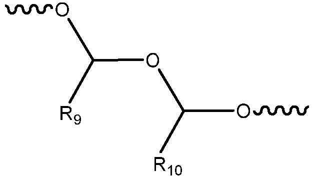 in which R₉ and R₁₀ independently represent an oxygen atom, an aliphatic hydrocarbon chain, optionally branched and/or unsaturated, a heteroaryl group independently comprising one or more oxygen and/or nitrogen atoms, an aldehyde function, a carboxylic acid group, a diol, a substituted diol, a group of formula -R₁₁-(X)ₙ-R₁₂ in which R₁₁ represents an aliphatic carbon chain C₁ to C₄ optionally branched and/or unsaturated, X represents a heteroatom chosen from oxygen and nitrogen, n is an integer from 1 to 4 and R₁₂ is a hydrogen atom, an optionally branched and/or unsaturated aliphatic hydrocarbon chain, a heteroaryl group independently comprising one or more oxygen and/or nitrogen atoms, an aldehyde function, a carboxylic acid group, a diol or a substituted diol.

5. Composition for use according to any one of claims 1 to 4, wherein the number of monomers "a" is such that the mass of the said polymers of formula (I) is greater than or equal to 3000 daltons.

6. Composition for use according to any one of claims 1 to 5, wherein rate of substitution "x" is between 10 and 150%.

7. Composition for use according to any one of the preceding claims, wherein rate of substitution "y" is between 10 and 170%.

8. A composition for its use according to any one of the preceding claims, wherein said biocompatible polymer further comprises functional chemical groups Z, different from X and Y, capable of conferring additional biological or physicochemical properties to said polymer.

9. Composition for its use according to claim 8, wherein rate of substitution "z" of all the monomers A by groups Z is between 1 and 50%.

10. Composition for its use according to claim 8 or 9, wherein the Z group is a substance capable of conferring on the said polymers an improved solubility or lipophilicity.

11. A composition for its use according to claim 10, wherein the Z groups are the same or different and are selected from the group consisting of amino acids, fatty acids, fatty alcohols, ceramides, or derivatives thereof, or nucleotide addressing sequences.

12. Composition for its use according to any one of claims 4 to 11 wherein the groups R₉ and R₁₀ are independently and optionally substituted with a group Z.
